(19) Europäisches Patentamt / European Patent Office / Office européen des brevets

(11) **EP 3 535 413 B1**

(12) **EUROPEAN PATENT SPECIFICATION**

(45) Date of publication and mention of the grant of the patent:
**28.01.2026   Bulletin 2026/05**

(21) Application number: **17808237.6**

(22) Date of filing: **02.11.2017**

(51) International Patent Classification (IPC):
***C12Q 1/68*** *(2018.01)*      ***C12Q 1/6883*** *(2018.01)*
***C12Q 1/6809*** *(2018.01)*

(52) Cooperative Patent Classification (CPC):
(C-Sets available)
**C12Q 1/6883; C12Q 1/6809;** C12Q 1/6851;
C12Q 2600/156                                      (Cont.)

(86) International application number:
**PCT/US2017/059808**

(87) International publication number:
**WO 2018/085603 (11.05.2018 Gazette 2018/19)**

(54) **METHODS FOR ASSESSING RISK USING TOTAL AND SPECIFIC CELL-FREE DNA**

RISIKOABSCHAETZUNG ANHAND VON GESAMTER UND SPEZIFISCHER AUSSERZELLULAERER DNA

PROCÉDÉS D'ÉVALUATION DU RISQUE À L'AIDE D'ADN ACELLULAIRE TOTAL ET SPÉCIFIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   02.11.2016   US 201662416689 P
                 29.04.2017   PCT/US2017/030293

(43) Date of publication of application:
**11.09.2019   Bulletin 2019/37**

(60) Divisional application:
**24198244.6 / 4 488 383**

(73) Proprietor: **The Medical College of Wisconsin, Inc.**
**Milwaukee, WI 53226 (US)**

(72) Inventors:
• **MITCHELL, Aoy, Tomita**
  **Elm Grove, WI 53122 (US)**
• **MITCHELL, Michael**
  **Elm Grove, WI 53122 (US)**
• **STAMM, Karl**
  **Wauwatosa, WI 53213 (US)**

(74) Representative: **Dolphin, Kirsty Mairi**
**Venner Shipley LLP**
**200 Aldersgate**
**London EC1A 4HD (GB)**

(56) References cited:
   WO-A1-2016/063122      WO-A1-2016/123698
   WO-A1-2016/176662      WO-A2-2014/194113

• **MARICA GRSKOVIC ET AL: "Validation of a Clinical-Grade Assay to Measure Donor-Derived Cell-Free DNA in Solid Organ Transplant Recipients", JOURNAL OF MOLECULAR DIAGNOSTICS,THE, vol. 18, no. 6, 7 October 2016 (2016-10-07), US, pages 890 - 902, XP055440489, ISSN: 1525-1578, DOI: 10.1016/ j.jmoldx.2016.07.003**
• **PAUL M. K. GORDON ET AL: "An Algorithm Measuring Donor Cell-Free DNA in Plasma of Cellular and Solid Organ Transplant Recipients That Does Not Require Donor or Recipient Genotyping", FRONTIERS IN CARDIOVASCULAR MEDICINE, vol. 3, 22 September 2016 (2016-09-22), XP055440493, DOI: 10.3389/fcvm.2016.00033**

- ADAMEK MARTINA ET AL: "A fast and simple method for detecting and quantifying donor-derived cell-free DNA in sera of solid organ transplant recipients as a biomarker for graft function", CLINICAL CHEMISTRY AND LABORATORY MEDICINE : JOURNAL OF THE FORUM OF THE EUROPEAN SOCIETIES OF CLINICAL CHEMISTRY,, vol. 54, no. 7, 1 July 2016 (2016-07-01), pages 1147 - 1155, XP009502752, ISSN: 1437-4331, [retrieved on 20151117], DOI: 10.1515/CCLM-2015-0622
- HIDESTRAND MATS ET AL: "Highly Sensitive Noninvasive Cardiac Transplant Rejection Monitoring Using Targeted Quantification of Donor-Specific Cell-Free Deoxyribonucleic Acid", JOURNAL OF THE AMERICAN COLLEGE OF CARDIOLOGY, vol. 63, no. 12, 1 April 2014 (2014-04-01), pages 1224 - 1226, XP028831507, ISSN: 0735-1097, DOI: 10.1016/J.JACC.2013.09.029
- BERGALLO MASSIMILIANO ET AL: "A novel TaqMAMA assay for allelic discrimination of TLR9 rs352140 polymorphism", JOURNAL OF VIROLOGICAL METHODS, vol. 243, 31 January 2017 (2017-01-31), pages 25 - 30, XP029939454, ISSN: 0166-0934, DOI: 10.1016/J.JVIROMET.2017.01.015

(52) Cooperative Patent Classification (CPC): (Cont.)

C-Sets
**C12Q 1/6809, C12Q 2537/165, C12Q 2545/114;** C12Q 1/6851, C12Q 2525/185, C12Q 2535/125, C12Q 2537/143

**Description**

**RELATED APPLICATIONS**

This application claims the benefit under 35 U.S.C. § 119(e), § 120, and § 365(c) of the filing date of U.S. Provisional Application No. 62/416,689, filed November 2, 2016 and International Application No. PCT/US2017/030293, filed April 29, 2017.

**FIELD OF THE INVENTION**

This invention relates to methods and compositions for assessing risk by measuring total and specific cell-free nucleic acids, such as cell-free DNA, in a subject. The methods and compositions provided herein can be used to determine risk of a condition, such as transplant rejection or other adverse transplant outcomes.

**BACKGROUND OF THE INVENTION**

The ability to detect and quantify low levels of nucleic acids in a sample may permit the early detection of a condition, such as transplant rejection or other adverse transplant outcomes. Current methods for quantitative analysis of nucleic acid populations, however, are limited. The Journal of Molecular Diagnostics (2016) 18(6) 890-902 provides a risk assessment of transplant recipients based on the relation of donor specific cfDNA to total cf DNA.

**SUMMARY OF INVENTION**

The scope of the invention is defined by the appended claims. In one aspect, a method of assessing one or more samples from a subject, comprising determining a value for the amount of total cell-free nucleic acids (such as DNA) in one or more samples from the subject, and determining a value for the amount of specific cell-free nucleic acids (such as DNA) in one or more samples from the subject is provided. In one embodiment, the method further comprises providing the value for the amount of total cell-free nucleic acids (such as DNA) in the one or more samples from the subject and the value for the amount of specific cell-free nucleic acids (such as DNA) in the one or more samples from the subject.

In one embodiment of any one of the methods provided herein, the value for the amount of total cell-free nucleic acids (such as DNA) and value for the amount of specific cell-free nucleic acids (such as DNA) are provided in a report.

In one embodiment of any one of the methods or reports provided herein, the value for the amount of total cell-free nucleic acids (such as DNA) and value for the amount of specific cell-free nucleic acids (such as DNA) is from one or more samples taken within 14 hours of a surgery. In one embodiment of any one of the methods or reports provided herein, the value for the amount of total cell-free nucleic acids (such as DNA) and value for the amount of specific cell-free nucleic acids (such as DNA) are from one or more samples taken within 24 hours of a surgery. In one embodiment of any one of the methods or reports provided herein, the surgery is a transplant surgery. In one embodiment of any one of the methods or reports provided herein, the value for the amount of total cell-free nucleic acids (such as DNA) and value for the amount of specific cell-free nucleic acids (such as DNA) is from one or more samples taken within 14 hours of cross-clamp removal. In one embodiment of any one of the methods or reports provided herein, the value for the amount of total cell-free nucleic acids (such as DNA) and value for the amount of specific cell-free nucleic acids (such as DNA) is from one or more samples taken within 24 hours of cross-clamp removal. In one embodiment of any one of the methods or reports provided herein, the cross-clamp removal is of a transplant, such as a heart transplant.

In one embodiment, any one of the methods provided can further comprise determining a value for the amount of total cell-free nucleic acids (such as DNA) in one or more other samples from the subject, and determining a value for the amount of specific cell-free nucleic acids (such as DNA) in one or more other samples from the subject, wherein the one or more other samples are from a subsequent time point. In one embodiment of any one of the methods or reports provided herein, the subsequent time point is at least one day later. In one embodiment of any one of the methods or reports provided herein, the subsequent time point is at least 1, 2, 3, 4, 5, or 6 days later. In one embodiment of any one of the methods or reports provided herein, the subsequent time point is at least one week later. In one embodiment of any one of the methods or reports provided herein, the subsequent time point is at least two weeks later. In one embodiment of any one of the methods or reports provided herein, the subsequent time point is a month later.

In one embodiment, any one of the methods provided herein can further comprise determining a value for the amount of total cell-free nucleic acids (such as DNA) in one or more further samples from the subject, and determining a value for the amount of specific

cell-free nucleic acids (such as DNA) in one or more further samples from the subject, wherein the one or more further samples are from one or more other subsequent time points. In one embodiment of any one of the methods or reports provided herein, the one or more other subsequent time points are at one-week intervals. In one embodiment of any one of the methods or reports provided herein, the one or more other subsequent time points are at two-week intervals. In one embodiment of any one of the methods or reports provided herein, the one or more other subsequent time points are at monthly intervals.

In one embodiment of any one of the methods provided herein, the values for the amounts of total cell-free nucleic acids (such as DNA) and values for the amounts of specific cell-free nucleic acids (such as DNA) are provided in a report. In one embodiment of any one of the methods or reports provided herein, the values for the amounts of total cell-free nucleic acids (such as DNA) and values for the amounts of specific cell-free nucleic acids (such as DNA) are provided separately in the report. In one embodiment of any one of the methods or reports provided herein, the values for the amounts of total cell-free nucleic acids (such as DNA) and values for the amounts of specific cell-free nucleic acids (such as DNA) are provided in separate graphs showing the values for the amounts of each over time. In one embodiment of any one of the methods or reports provided herein, the values for the amounts of total cell-free nucleic acids (such as DNA) and values for the amounts of specific cell-free nucleic acids (such as DNA) are provided together in the report. In one embodiment of any one of the methods or reports provided herein, the values for the amounts of total cell-free nucleic acids (such as DNA) and values for the amounts of specific cell-free nucleic acids (such as DNA) are provided in the same graph in the report. In one embodiment of any one of the methods or reports provided herein, the graph is a scatter plot. In one embodiment of any one of the methods or reports provided herein, the values for the amounts of total cell-free nucleic acids (such as DNA) and values for the amounts of specific cell-free nucleic acids (such as DNA) are provided separately and together in the report. In one embodiment of any one of the methods or reports provided herein, the values for the amounts of total cell-free nucleic acids (such as DNA) and values for the amounts of specific cell-free nucleic acids (such as DNA) are provided in separate graphs showing the values for the amounts of each over time and the values for the amounts of total cell-free nucleic acids (such as DNA) and values for the amounts of specific cell-free nucleic acids (such as DNA) are provided together in another graph in the report. In one embodiment of any one of the methods or reports provided herein, the another graph is a scatter plot.

The subject is a transplant recipient, such as a pediatric transplant recipient. In one embodiment of any one of the methods or reports provided herein, the transplant recipient is a cardiac transplant recipient. In one embodiment of any one of the methods or reports provided herein, the subject is a pediatric cardiac transplant recipient.

The specific cell-free nucleic acids (such as DNA) are donor-specific cell-free nucleic acids (such as DNA). In one embodiment of any one of the methods or reports provided herein, the total cell-free nucleic acids (such as DNA) are measured using PCR, such as real-time PCR. In one embodiment of any one of the methods or reports provided herein, the specific cell-free nucleic acids (such as DNA) are measured using a next-generation sequencing method or mismatch amplification-based quantitative method.

In one aspect, a method of assessing risk in a subject, comprising obtaining a value for the amount of total cell-free nucleic acids (such as DNA) in one or more samples from the subject, obtaining a value for the amount of specific cell-free nucleic acids (such as DNA) in one or more samples from the subject, and assessing the risk in the subject is provided.

Disclosed but not part of the invention, the method further comprises obtaining the one or more samples from the subject. In one embodiment of any one of the methods provided herein, the method further comprises providing the one or more samples from the subject.

In one embodiment of any one of the methods or reports provided herein, the one or more samples are from the subject within 14 hours of a surgery. In one embodiment of any one of the methods or reports provided herein, the one or more samples are from the subject within 24 hours of a surgery. In one embodiment of any one of the methods or reports provided herein, the surgery is a transplant surgery. In one embodiment of any one of the methods or reports provided herein, the one or more samples are from the subject within 14 hours of cross-clamp removal. In one embodiment of any one of the methods or reports provided herein, the one or more samples are from the subject within 24 hours of cross-clamp removal. In one embodiment of any one of the methods or reports provided, the cross-clamp removal is of a transplant surgery, such as a heart transplant surgery.

In one embodiment of any one of the methods provided herein, the value for the amount of total cell-free nucleic acids (such as DNA) and value for the amount of specific cell-free nucleic acids (such as DNA) are obtained from a report.

In one embodiment of any one of the methods provided herein, the method further comprises obtaining a value for the amount of total cell-free nucleic acids (such as DNA) in one or more other samples from the subject, and obtaining a value for the amount of specific cell-free nucleic acids (such as DNA) in one or more other samples from the subject, wherein the one or more other samples are from a subsequent time point.

In one embodiment of any one of the methods provided herein, the method further comprises obtaining and/or providing the one or more other samples from the subject. In one embodiment of any one of the methods or reports provided herein, the subsequent time point is at least one day later. In one embodiment of any one of the methods or reports provided herein, the subsequent time point is at least 1, 2, 3, 4, 5, or 6 days later. In one embodiment of any one of the methods or reports provided herein, the subsequent time point is at least one week later. In one embodiment of any one of the methods or reports provided herein, the subsequent time point is at least two weeks later. In one embodiment of any one of the methods or reports provided herein, the subsequent time point is a month later.

In one embodiment of anyone of the methods provided herein, the method further comprises obtaining a value for the amount of total cell-free nucleic acids (such as DNA) in one or more further samples from the subject, and obtaining a value for the amount of specific cell-free nucleic acids (such as DNA) in one or more further samples from the subject, wherein the one or more further samples are from one or more other subsequent time points.

Disclosed but not part of the invention is a method that further comprises obtaining and/or providing the one or more further samples from the subject.

In one embodiment of any one of the methods or reports provided herein, the one or more other subsequent time points are at one-week intervals. In one embodiment of any one of the methods or reports provided herein, the one or more other subsequent time points are at two-week intervals. In one embodiment of any one of the methods or reports provided herein, the one or more other subsequent time points are at monthly intervals.

In one embodiment of any one of the methods provided herein, the values for the amounts of total cell-free nucleic acids (such as DNA) and the values for the amounts of specific cell-free nucleic acids (such as DNA) are obtained from a report. In one embodiment of any one of the methods or reports provided herein, the values for the amounts of total cell-free nucleic acids (such as DNA) and values for the amounts of specific cell-free nucleic acids (such as DNA) are provided separately in the report. In one embodiment of any one of the methods or reports provided herein, the values for the amounts of total cell-free nucleic acids (such as DNA) and values for the amounts of specific cell-free nucleic acids (such as DNA) are provided in separate graphs showing the values for the amounts of each over time. In one embodiment of any one of the methods or reports provided herein, the values for the amounts of total cell-free nucleic acids (such as DNA) and values for the amounts of specific cell-free nucleic acids (such as DNA) are provided together in the report. In one embodiment of any one of the methods or reports provided herein, the values for the amounts of total cell-free nucleic acids (such as DNA) and values for the amounts of specific cell-free nucleic acids (such as DNA) are provided in the same graph in the report. In one embodiment of any one of the methods or reports provided herein, the graph is a scatter plot. In one embodiment of any one of the methods or reports provided herein, the values for the amounts of total cell-free nucleic acids (such as DNA) and values for the amounts of specific cell-free nucleic acids (such as DNA) are provided separately and together in the report. In one embodiment of any one of the methods or reports provided herein, the values for the amounts of total cell-free nucleic acids (such as DNA) and values for the amounts of specific cell-free nucleic acids (such as DNA) are provided in separate graphs showing the values for the amounts of each over time and the values for the amounts of total cell-free nucleic acids (such as DNA) and values for the amounts of specific cell-free nucleic acids (such as DNA) are provided together in another graph in the report. In one embodiment of any one of the methods or reports provided herein, the another graph is a scatter plot.

In one embodiment of any one of the methods or reports provided herein, the report or graph(s) provided therein comprise an indicator of one or more threshold values. In one embodiment of any one of the methods or reports provided herein, the threshold value for donor-specific cell-free nucleic acids (such as DNA) indicated in the report or graph thereof is 0.8%, 0.9%, 1%, 1.1%, 1.2%, 1.3% or more. In one embodiment of any one of the methods or reports provided herein, the threshold value for total cell-free nucleic acids (such as DNA) indicated in the report or graph thereof is 15, 16, 17, 18, 19, 20, 21, 22, 23, 24, 25, 30, 35, 40, 45, 50 ng/mL plasma or more. In one embodiment of any one of the methods or reports provided herein, the threshold value for donor-specific cell-free nucleic acids (such as DNA) indicated in the report or graph thereof is 0.8%, 0.9%, 1%, 1.1%, 1.2%, 1.3% or more and the threshold value for total cell-free nucleic acids (such as DNA) indicated in the report or graph thereof is 15, 16, 17, 18, 19, 20, 21, 22, 23, 24, 25, 30, 35, 40, 45, 50 ng/mL plasma or more. In one embodiment of any one of the methods or reports provided herein, the threshold value for donor-specific cell-free nucleic acids (such as DNA) indicated in the report or graph thereof is 0.8% and the threshold value for total cell-free nucleic acids (such as DNA) indicated in the report or graph thereof is 15 ng/mL plasma.

In one embodiment of any one of the methods or reports provided herein, the subject is a transplant recipient. In one embodiment of any one of the methods or reports provided herein, the transplant recipient is a cardiac transplant recipient. In one embodiment of any one of the methods or reports provided herein, the subject is a pediatric cardiac transplant recipient.

In one embodiment of any one of the methods or reports provided herein, the specific cell-free nucleic acids (such as DNA) is donor-specific cell-free nucleic acids (such as DNA).

In one embodiment of any one of the methods or reports provided herein, when the value for the amount of total cell-free nucleic acids (such as DNA) is above a threshold value and the value for the amount of specific cell-free nucleic acids (such as DNA) is above a threshold value, rejection or other adverse outcome or poor prognosis (such as death) in the transplant subject, is indicated. In one embodiment of any one of the methods provided herein, when the value for the amount of total cell-free nucleic acids (such as DNA) is above a threshold value and the value for the amount of specific cell-free nucleic acids (such as DNA) is above a threshold value, a therapy, such as anti-rejection treatment, is suggested to the subject and/or further or increased monitoring of the subject is performed or suggested. It is disclosed but not part of the claimed invention that the anti-rejection treatment comprises a steroid and/or admission to a hospital. In one embodiment of any one of the methods or reports provided herein, when the value for the amount of total cell-free nucleic acids (such as DNA) is above a threshold value and the value for the amount of specific cell-free nucleic acids (such as DNA) is below a threshold value, infection is indicated. In one embodiment of any one of the methods provided herein, when the value is for the amount of total cell-free nucleic acids (such as DNA) is above a threshold value and the value for the amount of specific cell-free nucleic acids (such as DNA) is below a threshold value, anti-infection treatment is administered or suggested to the subject and/or further or increased monitoring of the subject is performed or suggested. In one embodiment of any one of the methods provided herein, the anti-infection treatment comprises an antibiotic or a reduction or change in an immunosuppressive therapy. In one embodiment of any one of the methods or reports provided herein, when the value for the amount of total cell-free nucleic acids (such as DNA) is below a threshold value and the value for the amount of specific cell-free nucleic acids (such as DNA) is above a threshold value, early stage rejection is indicated. In one embodiment of any one of the methods provided herein, when the value for the amount of total cell-free nucleic acids (such as DNA) is below a threshold value and the value for the amount of specific cell-free nucleic acids (such as DNA) is above a threshold value, anti-rejection treatment is suggested to the subject and/or further or increased monitoring of the subject is performed or suggested. It is disclosed but not part of the invention that the anti-rejection treatment comprises a steroid. In one embodiment of any one of the methods provided herein, the anti-rejection treatment does not comprise admission to a hospital. In one embodiment of any one of the methods or reports provided herein, when the value for the amount of total cell-free nucleic acids (such as DNA) is below a threshold value and the value for the amount of specific cell-free nucleic acids (such as DNA) is below a threshold value, no clinical condition is indicated. In one embodiment of any one of the methods provided herein, when the value for the amount of total cell-free nucleic acids (such as DNA) is below a threshold value and the value for the amount of specific cell-free nucleic acids (such as DNA) is below a threshold value, further or decreased monitoring of the subject is performed or suggested.

In one embodiment of any one of the methods provided herein, the monitoring of the subject comprises any one of the methods provided herein.

The sample comprises blood, plasma or serum.

In one aspect, a report comprising any one or more of the values provided herein is provided. In one embodiment, the report comprises a value for the amount of total cell-free nucleic acids (such as DNA) in one or more samples from a subject, and a value for the amount of specific cell-free nucleic acids (such as DNA) in one or more samples from the subject. In one embodiment of any one of the methods or reports provided, the value for the amount of total cell-free nucleic acids (such as DNA) and value for the amount of specific cell-free nucleic acids (such as DNA) is from one or more samples taken within 14 hours of a surgery. In one embodiment of any one of the methods or reports provided, the value for the amount of total cell-free nucleic acids (such as DNA) and value for the amount of specific cell-free nucleic acids (such as DNA) is from one or more samples taken within 24 hours of a transplant surgery. In one embodiment of any one of the methods or reports provided, the value for the amount of total cell-free nucleic acids (such as DNA) and value for the amount of specific cell-free nucleic acids (such as DNA) is from one or more samples taken within 14 hours of cross-clamp removal. In one embodiment of any one of the methods or reports provided, the value for the amount of total cell-free nucleic acids (such as DNA) and value for the amount of specific cell-free nucleic acids (such as DNA) is from one or more samples taken within 24 hours of cross-clamp removal.

In one embodiment of any one of the reports provided, the report further comprises a value for the amount of total cell-free nucleic acids (such as DNA) from one or more other samples from the subject, and a value for the amount of specific cell-free nucleic acids (such as DNA) from one or more other samples from the subject, wherein the one or more other samples are from a different, such as subsequent, time point. In one embodiment of any one of the methods or reports provided herein, the subsequent time point is at least one day later. In one embodiment of any one of the methods or reports provided herein, the subsequent time point is at least 1, 2, 3, 4, 5, or 6 days later. In one embodiment of any one of the methods or reports provided, the subsequent time point is at least one week later. In one embodiment of any one of the methods or reports provided, the subsequent time point is at least two weeks later. In one embodiment of any one of the methods or reports provided, the subsequent time point is a month later.

In one embodiment of any one of the reports provided, the report further comprises a value for the amount of total cell-free nucleic acids (such as DNA) from one or more further samples from the subject, and a value for the amount of specific cell-free nucleic acids (such as DNA) from one or more further samples from the subject, wherein the one or more further samples are from one or more other time points, such as subsequent time points. In one embodiment of any one of the methods or reports provided, the one or more other subsequent time points are at one-week intervals. In one embodiment of any one of the methods or reports provided, the one or more other subsequent time points are at two-week intervals. In one embodiment of any one of the methods or reports provided, the one or more other subsequent time points are at monthly intervals.

In one embodiment of any one of the methods or reports provided, a baseline amount of total cell-free nucleic acids (such as DNA) from the subject, such as before the surgery or some other earlier time point, and a value for the amount of specific cell-free nucleic acids (such as DNA) from the subject, such as before the surgery or some other earlier time point, is obtained by any one of the methods provided herein and/or provided, such as in any one of the reports provided.

In one embodiment of any one of the methods or reports provided, the values for the amounts of total cell-free nucleic acids (such as DNA) and values for the amounts of specific cell-free nucleic acids (such as DNA) are provided separately in the report. In one embodiment of any one of the methods or reports provided, the values for the amounts of total cell-free nucleic acids (such as DNA) and values for the amounts of specific cell-free nucleic acids (such as DNA) are provided in separate graphs showing the values for the amounts of each over time. In one embodiment of any one of the methods or reports provided, the values for the amounts of total cell-free nucleic acids (such as DNA) and values for the amounts of specific cell-free nucleic acids (such as DNA) are provided together in the report. In one embodiment of any one of the methods or reports provided, the values for the amounts of total cell-free nucleic acids (such as DNA) and values for the amounts of specific cell-free nucleic acids (such as DNA) are provided in the same graph in the report. In one embodiment of any one of the methods or reports provided, the graph is a scatter plot. In one embodiment of any one of the methods or reports provided, the values for the amounts of total cell-free nucleic acids (such as DNA) and values for the amounts of specific cell-free nucleic acids (such as DNA) are provided separately and together in the report. In one embodiment of any one of the methods or reports provided, the values for the amounts of total cell-free nucleic acids (such as DNA) and values for the amounts of specific cell-free nucleic acids (such as DNA) are provided in separate graphs showing the values for the amounts of each over time and the values for the amounts of total cell-free nucleic acids (such as DNA) and values for the amounts of specific cell-free nucleic acids (such as DNA) are provided together in another graph in the report. In one embodiment of any one of the methods or reports provided, the another graph is a scatter plot.

In one embodiment of any one of the methods or reports provided, the subject is a transplant recipient. In one embodiment of any one of the methods or reports provided, the transplant recipient is a cardiac transplant recipient. In one embodiment of any one of the methods or reports provided, the subject is a pediatric cardiac transplant recipient.

In one embodiment of any one of the methods or reports provided, the specific cell-free nucleic acids (such as DNA) is donor-specific cell-free nucleic acids (such as DNA).

In one embodiment of any one of the methods or reports provided, the total cell-free nucleic acids (such as DNA) is measured using PCR, such as real-time PCR.

In one embodiment of any one of the methods or reports provided, the specific cell-free nucleic acids (such as DNA) are measured using a next-generation sequencing method or mismatch amplification-based quantitative method.

In one embodiment of any one of the methods or reports provided herein, the mismatch amplification-based quantitative method is any one of such methods provided herein. In one embodiment of any one of the methods or reports provided herein, such a mismatch method comprises, for each of a plurality of single nucleotide variant (SNV) targets, obtaining results from nucleic acid amplification, such as by polymerase chain reaction (PCR), on a sample, or portion thereof, with at least one primer pair, wherein the at least one primer pair comprises a forward primer and a reverse primer, wherein the at least one primer pair comprises a primer with a 3' mismatch (e.g., penultimate mismatch) relative to one sequence (e.g., allele) of the SNV target but a 3' double mismatch relative to another sequence (e.g., allele) of the SNV target and specifically amplifies the one sequence (e.g., allele) of the SNV target.

In one embodiment of any one of the methods or reports provided herein, such a mismatch method further comprises, for each SNV target, obtaining results from a quantification assay with at least one another primer pair, wherein the at least one another primer pair comprises a forward primer and a reverse primer, wherein the at least one another primer pair specifically amplifies another sequence (e.g., allele) of the SNV target.

In one embodiment of any one of the methods or reports provided herein, such a mismatch method comprises, for each of a plurality of single nucleotide variant (SNV) targets, performing nucleic acid amplification, such as by PCR, on a sample, or portion thereof, with at least two primer pairs, wherein each primer pair comprises a forward primer and a reverse primer, wherein one of the at least two primer pairs comprises a 3' mismatch (e.g., penultimate) relative to one sequence (e.g., allele) of the SNV target but a 3' double mismatch relative to another sequence (e.g., allele) of the SNV target and specifically amplifies the one sequence (e.g., allele) of the SNV target, and another of the at least two primer pairs specifically amplifies the another sequence (e.g., allele) of the SNV target.

In one embodiment of any one of the methods or reports provided herein, such a mismatch method comprises obtaining results from nucleic acid amplification, such as by PCR, for each of a plurality of single nucleotide variant (SNV) targets, performed on a sample, or portion thereof, with at least two primer pairs, wherein each primer pair comprises a forward primer and a reverse primer, wherein one of the at least two primer pairs comprises a 3' mismatch (e.g., penultimate) relative to one sequence (e.g., allele) of the SNV target but a 3' double mismatch relative to another sequence (e.g., allele) of the SNV target and specifically amplifies the one sequence (e.g., allele) of the SNV target, and another of the at least two primer pairs specifically amplifies the another sequence (e.g., allele) of the SNV target.

In one embodiment of any one of the methods or reports provided herein, such a mismatch method comprises obtaining results from nucleic acid amplifications, such as PCR on a sample with at least one primer pair as provided herein, such as at least two primer pairs, wherein each primer pair comprises a forward primer and a reverse primer, selecting informative results based on the genotype of the specific nucleic acids and/or non-specific nucleic acids, and determining the amount of the non-specific nucleic acids in the sample based on the informative results. In one embodiment of any one of the methods or reports provided herein, such a mismatch method further comprises identifying the plurality of SNV targets. In one embodiment of any one of the methods or reports provided herein, such a mismatch method further comprises inferring the genotype of the non-specific nucleic acids.

In one embodiment of any one of the methods or reports provided herein, such a mismatch method comprises obtaining results from 1) nucleic acid amplification, such as PCR, for each of a plurality of SNV targets, performed on a sample, or portion thereof, with at least one primer pair, such as at least two primer pairs, wherein each primer pair comprises a forward primer and a reverse primer, wherein one of the at least one, such as at least two, primer pair, comprises a 3' mismatch (e.g., penultimate) relative to one sequence (e.g., allele) of the SNV target but a 3' double mismatch relative to another sequence (e.g., allele) of the SNV target and specifically amplifies the one sequence (e.g., allele) of the SNV target and 2) a determination of informative results based on the specific genotype and/or a prediction of the likely non-specific genotype. In one embodiment of any one of such mismatch methods, when there are at least two primer pairs, the another primer pair specifically amplifies the another sequence (e.g., allele) of each SNV target and quantification results are obtained with the another primer pair for each of the SNV targets.

In one embodiment of any one of the methods or reports provided herein, such a mismatch method comprises obtaining results from 1) nucleic acid amplifications, such as PCR, for each of a plurality of SNV targets, performed on a sample, or portion thereof, with at least two primer pairs, wherein each primer pair comprises a forward primer and a reverse primer, wherein one of the at least two primer pairs comprises a 3' mismatch (e.g., penultimate) relative to one sequence (e.g., allele) of the SNV target but a 3' double mismatch relative to another sequence (e.g., allele) of the SNV target and specifically amplifies the one sequence (e.g., allele) of the SNV target, and another of the at least two primer pairs specifically amplifies the another sequence (e.g., allele) of the SNV target, and 2) a determination of informative results based on the specific genotype and/or a prediction of the likely non-specific genotype.

In one embodiment of any one of the methods or reports provided herein, such a mismatch method further comprises at least one another primer pair for each SNV target and/or obtaining results with nucleic acid amplification, such as PCR, therewith. In one embodiment of any one of such mismatch methods, the at least one another primer pair comprises a 3' mismatch (e.g., penultimate) relative to another sequence (e.g., allele) of the SNV target but a 3' double mismatch relative to the one sequence (e.g., allele) of the SNV target and specifically amplifies the another sequence (e.g., allele) of the SNV target.

In one embodiment of any one of the methods or reports provided herein, such a mismatch method further comprises assessing the amount of specific nucleic acids based on the results.

In one embodiment of any one of such mismatch methods, the results are informative results.

In one embodiment of any one of such mismatch methods, the method further comprises selecting informative results of the amplifications, such as PCR amplifications. In one embodiment of any one of such mismatch methods, the selected informative results are averaged, such as a median average. In one embodiment of any one of such mismatch methods, the results can be further analyzed with Robust Statistics. In one embodiment of any one of such mismatch methods, the results can be further analyzed with a Standard Deviation, such as a Robust Standard Deviation, and/or Coefficient of Variation, such as a Robust Coefficient of Variation, or % Coefficient of Variation, such as a % Robust Coefficient of Variation.

In one embodiment of any one of such mismatch methods, the informative results of the nucleic acid amplifications, such as PCR, are selected based on the genotype of the non-specific nucleic acids and/or specific nucleic acids.

In one embodiment of any one of such mismatch methods, the method further comprises obtaining the genotype of the non-specific nucleic acids and/or specific nucleic acids.

In one embodiment of any one of such mismatch methods, the method further comprises selecting informative results based on the specific genotype and/or prediction of the likely non-specific genotype. In one embodiment of any one of such mismatch methods, when the genotype of the non-specific nucleic acids is not known or obtained, the method further comprises assessing results based on a prediction of the likely non-specific genotype. In one embodiment of any one of such mismatch methods, the method comprises the amount of the non-specific nucleic acids in the sample based on the informative results and prediction. In one embodiment of any one of such mismatch methods, the assessing or prediction is performed with an expectation-maximization algorithm. In one embodiment of any one of such mismatch methods, expectation-maximization is used to predict the likely non-specific genotype.

In one embodiment of any one of such mismatch methods, maximum likelihood is used to calculate the amount of non-specific nucleic acids.

In one embodiment of any one of the methods or reports provided herein, the amount of the specific cell-free nucleic acids (such as DNA) is the ratio or percentage of specific nucleic acids to total or non-specific nucleic acids.

In one embodiment of any one of such mismatch methods, there is at least one primer pair, at least two primer pairs, at least three primer pairs, at least four primer pairs or more per SNV target. In one embodiment of any one of such mismatch methods, the plurality of SNV targets is at least 45, 48, 50, 55, 60, 65, 70, 75, 80, 85 or 90 or more. In one embodiment of any one of such mismatch methods, the plurality of SNV targets is at least 90, 95 or more targets. In one embodiment of any one of such mismatch methods, the plurality of SNV targets is less than 90, 95 or more targets. In one embodiment of any one of such mismatch methods, the plurality of SNV targets is less than 105 or 100 targets.

In one embodiment of any one of such mismatch methods, the mismatched primer(s) is/are the forward primer(s).

In one embodiment of any one of such mismatch methods, the reverse primers for the primer pairs for each SNV target is the same.

In one embodiment of any one of such mismatch methods, the method further comprises extracting nucleic acids from the sample.

In one embodiment of any one of such mismatch methods, the method further comprises an additional amplification step. In one embodiment of any one of such mismatch methods, the amplification is performed prior to the amplifications for quantification with mismatch primers.

In one embodiment, any one of the embodiments for the methods provided herein can be an embodiment for any one of the reports provided. In one embodiment, any one of the embodiments for the reports provided herein can be an embodiment for any one of the methods provided herein.

## BRIEF DESCRIPTION OF DRAWINGS

The accompanying drawings are not intended to be drawn to scale. The figures are illustrative only and are not required for enablement of the disclosure.

**Fig. 1** provides an exemplary, non-limiting diagram of "MOMA" primers that can be used in a mismatched amplification-based quantitative assays, such as those of PCT Application No. PCT/US2016/030313 In a polymerase chain reaction (PCR) assay, extension of the sequence containing SNV A is expected to occur, resulting in the detection of SNV A, which may be subsequently quantified. Extension of the SNV B, however, is not expected to occur due to the double mismatch.

**Fig. 2** shows results from a reconstruction experiment demonstrating the ability to use a mismatch amplification method to measure cell-free DNA.

**Fig. 3** provides the percent cell-free DNA measured with plasma samples from transplant recipient patients with a mismatch amplification method. All data comes from patients who have had biopsies. Dark points denote rejection.

**Fig. 4** provides further data from a mismatch amplification method on plasma samples. After transplant surgery, the donor percent levels drop off.

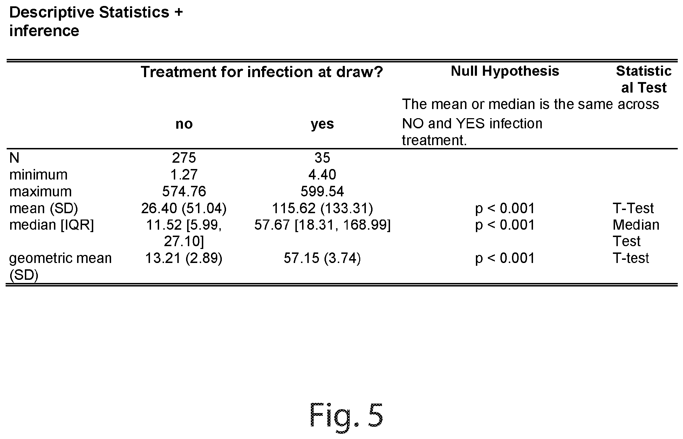

**Fig. 5** provides data of total cell-free DNA measurement in subjects that had or did not have infection treatment at blood draw.

**Fig. 6** provides two separate graphs showing an example of how values for total cell-free nucleic acids (such as DNA) and values for specific cell-free nucleic acids (such as DNA) over time can be displayed in a report.

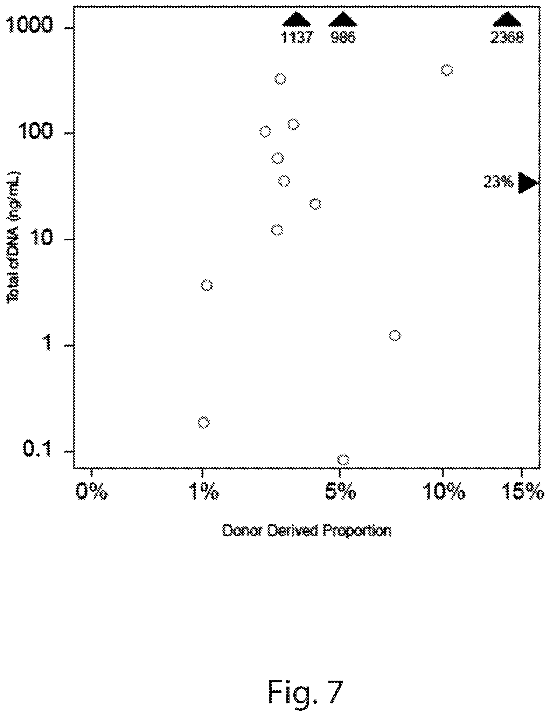

**Fig. 7** provides a graph (scatter plot) showing an example of how values for total cell-free nucleic acids (such as DNA) and values for specific cell-free nucleic acids (such as DNA) can be displayed together for various time points in a report. It is expected that when both the values for the total cell-free DNA and specific cell-free DNA are considered high, rejection is likely occurring and driving an inflammatory process or an adverse outcome or prognosis for a transplant subject is indicated. When the total cell-free DNA is considered high but specific cell-free DNA is not, there is likely to be an infection occurring in the subject but not rejection. However, when the total cell-free DNA is considered low along with what is considered high specific cell-free DNA, it is likely the subject is in an early stage of rejection and would otherwise be considered asymptomatic. Anti-rejection treatment could still be needed. Finally, when both total cell-free DNA and specific cell-free DNA are considered low, it is expected that no treatment is warranted, although it may still be necessary to continue monitoring the patient.

**Fig. 8** illustrates an example of a computer system with which some embodiments may operate.

**Fig. 9** provides a graph (scatter plot) showing an example of how values for total cell-free nucleic acids (such as DNA) and values for specific cell-free nucleic acids (such as DNA) can be displayed together in a report. In this example, 68 samples were analyzed; the triangles indicate outcomes of death.

## DETAILED DESCRIPTION OF THE INVENTION

Aspects of the disclosure relate to methods for assessing risk in a subject. The risk can be assessed in some embodiments using a combination of one or more values for the amount of total cell-free nucleic acids (such as DNA) and one or more values for the amount of specific cell-free nucleic acids (such as DNA) in a subject. Methods provided herein or otherwise known in the art can be used multiple times to obtain such values over time. Also included are reports that can include one or more of these values. Such reports can provide valuable information to a clinician. In some embodiments, the clinician can then assess a risk in the subject.

A "risk" as provided herein, refers to the presence or absence of any undesirable condition in a subject (such as a transplant recipient), or an increased likelihood of the presence or absence of such a condition, e.g., transplant rejection, infection. As provided herein, "increased risk" refers to the presence of any undesirable condition in a subject or an increased likelihood of the presence of such a condition. As provided herein, "decreased risk" refers to the absence of any undesirable condition in a subject or a decreased likelihood of the presence (or increased likelihood of the absence) of such a condition. As an example, early detection of rejection following implantation of a transplant (e.g., a heart transplant) can facilitate treatment and improve clinical outcomes. Transplant rejection remains a major cause of graft failure and late mortality and generally requires lifelong surveillance monitoring. Treatment of transplant rejections with immunosuppressive therapy has been shown to improve treatment outcomes, particularly if rejection is detected early.

Accordingly, in some embodiments of any one of the methods provided, the subject is a recipient of a transplant, and the risk is a risk associated with the transplant. In some embodiments of any one of the methods provided, the risk associated with the transplant is risk of transplant rejection, an anatomical problem with the transplant or injury to the transplant, infection, or other adverse outcome or prognosis in the subject. In some embodiments of any one of the methods provided, the injury to the transplant is initial or ongoing injury. In some embodiments of any one of the methods provided, the risk associated with the transplant is an acute condition or a chronic condition. In some embodiments of any one of the methods provided, the acute condition is transplant rejection including cellular rejection or antibody-mediated rejection. In some embodiments of any one of the methods provided, the chronic condition is graft vasculopathy. In some embodiments of any one of the methods provided, the risk associated with the transplant is indicative of the severity of the injury.

As used herein, "transplant" refers to the moving of an organ from a donor to a recipient for the purpose of replacing the recipient's damaged or absent organ. The transplant may be of one organ or more than one organ. In some embodiments, the term "transplant" refers to a transplanted organ or organs, and such meaning will be clear from the context the term is used. Examples of organs that can be transplanted include, but are not limited to, the heart, kidney(s), kidney, liver, lung(s), pancreas, intestine, etc. Any one of the methods or reports provided herein may be used regarding samples from a subject that has undergone a transplant of any one or more of the organs provided herein.

As used herein, the sample from a subject can be a biological sample. Examples of such biological samples include whole blood, plasma, serum, etc.

In some aspects, the methods include steps for determining a value for the amount of total cell-free nucleic acids (such as DNA) and a value for the amount of specific cell-free nucleic acids (such as DNA). As used herein, a "value" is any indicator that conveys information about an "amount". The indicator can be an absolute or relative value for the amount. As used herein, "amount" refers to the quantity of cell-free nucleic acids (such as DNA). Further, the value can be the amount, frequency, ratio, percentage, etc.

In some instances the values can be compared to a "threshold value". As used herein, a "threshold value" refers to any predetermined level or range of levels that is indicative of a state, the presence or absence of a condition or the presence or absence of a risk. The threshold value can take a variety of forms. It can be single cut-off value, such as a median or mean. It can be established based upon comparative groups, such as where the risk in one defined group is double the risk in another defined group. It can be a range, for example, where the tested population is divided equally (or unequally) into groups, such as a low-risk group, a medium-risk group and a high-risk group, or into quadrants, the lowest quadrant being subjects with the lowest risk and the highest quadrant being subjects with the highest risk. The threshold value can depend upon the particular population selected. For example, an apparently healthy population will have a different 'normal' range. As another example, a threshold value can be determined from baseline values before the presence of a state, condition or risk or after a course of treatment. Such a baseline can be indicative of a normal or other state in the subject not correlated with the risk or condition that is being tested for. In some embodiments, the threshold value can be a baseline value of the subject being tested. Accordingly, the predetermined values selected may take into account the category in which the subject falls. Appropriate ranges and categories can be selected with no more than routine experimentation by those of ordinary skill in the art.

In some embodiments of any one of the methods provided herein in regard to a heart transplant recipient, such threshold for donor-specific cell-free DNA is equal to or greater than 0.8%, 0.9%, or 1%, wherein a level above, respectively, is indicative of an increased risk and wherein a level at or below is indicative of a decreased risk. In some embodiments of any one of the methods provided herein in regard to a heart transplant recipient, such threshold is equal greater than 1.1%, 1.2% or 1.3%, wherein a level above, respectively, is indicative of an increased risk and wherein a level at or below is indicative of a decreased risk.

In some embodiments of any one of the methods provided herein, such as in regard to a risk, such as an infection or other undesirable outcome for a transplant subject, such threshold for total cell-free DNA may be greater than or equal to 15, 20, 25, 30, 35, 40, 45, or 50 ng/mL, such as per mL plasma. In some embodiments of any one of the methods provided herein, such threshold for total cell-free DNA may be greater than or equal to 50 ng/mL plasma. In some embodiments of any one of the methods provided herein, such threshold for total cell-free DNA may be greater than or equal to 100 ng/mL plasma. In some embodiments of any one of the methods provided herein, such threshold for total cell-free DNA may be in genome equivalents/mL, such as per mL plasma. In some embodiments of any one of the methods provided herein, such as in regard to a risk, such as an infection, such threshold for total cell-free DNA may be 2,800 genome equivalents/mL, such as per mL plasma. In some embodiments of any one of the methods provided herein, such as in regard to a risk, such as for sepsis, such threshold for total cell-free DNA may be 14,000 genome equivalents/mL, such as per mL plasma.

As used herein, "specific cell-free nucleic acids" refers to a subset of cell-free nucleic acids (such as DNA) that is within total cell-free nucleic acids (such as DNA) generally at a low level. In some embodiments, the specific cell-free nucleic acids (such as DNA) are cell-free nucleic acids (such as DNA) that are donor-specific (DS). DS cf-DNA refers to DNA that presumably is shed from the transplanted organ, the sequence of which matches (in whole or in part) the genotype of the donor who donated the transplanted organ. As used herein, "DS cf-DNA" may refer to a certain sequence or certain sequences in the DS cf-DNA population, where the sequence is distinguishable from the recipient cf-DNA (e.g., having a different sequence at a particular nucleotide location(s)), or it may refer to the entire DS cf-DNA population. "Total cell-free nucleic acids" are the total of a type of nucleic acids (such as DNA) that is present outside of a cell, e.g., in the blood, plasma, serum, etc. of a subject. Without wishing to be bound by any particular theory or mechanism, it is believed that such nucleic acids are released from cells, e.g., via apoptosis of the cells. In some embodiments of any one of the methods or reports provided herein, "specific cell-free nucleic acids" refers to those that are not native to a subject or are not wild-type. Non-specific cell-free nucleic acids are those that are native to a subject or are wild-type in such instances.

The values for the amount(s) of cell-free nucleic acids (such as DNA) can be "obtained" by any one of the methods provided herein, and the obtaining step(s) can include any one of the methods provided herein. "Obtaining" as used herein refers to any method by which the respective information or materials can be acquired. Thus, the respective information or materials can be acquired by experimental methods. Respective materials can be created, designed, etc. with various experimental or laboratory methods, in some embodiments. The respective information or materials can also be acquired by being given or provided with the information, such as in a report, or materials. Materials may be given or provided through commercial means (i.e. by purchasing), in some embodiments.

As provided herein, the risk can be determined using a combination of one or more values for the amount of total cell-free nucleic acids (such as DNA) as well as one or more values for the amount of specific cell-free nucleic acids (such as DNA) in one or more samples from a subject. Such methods, in some embodiments, further includes assessing a risk associated with the transplant in the subject based on the combination of values. In some embodiments, any one of the methods provided herein can comprise correlating an increase in a value for an amount of the total and/or specific cell-free nucleic acids (such as DNA) with an increased risk of a condition, such as transplant rejection. In some embodiments of any one of the methods provided herein, correlating comprises comparing a level (e.g., concentration, ratio or percentage) of total and specific cell-free nucleic acids (such as DNA) to a threshold value to identify a subject at increased or decreased risk.

For example, when the value for the amount of total cell-free DNA is above a threshold value and the value for the amount of specific cell-free DNA is above a threshold value, rejection or other adverse outcome or prognosis is indicated. As another example, when the value for the amount of total cell-free DNA is above a threshold value and the value for the amount of specific cell-free DNA is below a threshold value, infection is indicated. As a further example, when the value for the amount of total cell-free DNA is below a threshold value and the value for the amount of specific cell-free DNA is above a threshold value, early stage or asymptomatic rejection is indicated. As yet another example, when the value for the amount of total cell-free DNA is below a threshold value and the value for the amount of specific cell-free DNA is below a threshold value, no clinical condition is indicated, and further monitoring of the subject may or may not be performed or suggested to the subject.

Thus, changes in the levels of can also be monitored over time. In organ transplantation, this can form the basis of an individualized non-invasive screening test for rejection or a risk of a condition associated thereto. In one embodiment of any one of the methods provided herein, the method may further include an additional test(s) for assessing a condition, such as transplant rejection, transplant injury, etc. The additional test(s) may be any one of the methods provided herein. In some embodiments, for transplant recipients, the additional test is a determination of the value for the amount of total cell-free nucleic acids (such as DNA) and specific cell-free nucleic acids (such as DNA) in a sample from the subject. Further, such methods can aid in the selection, administration and/or monitoring of a treatment or therapy.

Treatment may be indicated. Thus, any one of the methods provided herein may include one or more steps of treatment or suggesting treatment to a subject (including providing information about the treatment to the subject, in some embodiments). For example, where rejection is indicated, anti-rejection therapies can be administered. "Administering" or "administration" or "administer" or the like means providing a material to a subject in a manner that is pharmacologically useful directly or indirectly. Thus, the term includes directing, such as prescribing, the subject or another party to administer the material. Administration of a treatment or therapy may be accomplished by any method known in the art (see, e.g., Harrison's Principle of Internal Medicine, McGraw Hill Inc.). Preferably, administration of a treatment or therapy occurs in a therapeutically effective amount. Compositions for different routes of administration are known in the art (see, e.g., Remington's Pharmaceutical Sciences by E. W. Martin).

In some embodiments of any one of the methods provided herein, the method may further comprise determining a treatment regimen based on the amounts. "Determining a treatment regimen", as used herein, refers to the determination of a course of action for the treatment of the subject. In one embodiment of any one of the methods provided herein, determining a treatment regimen includes determining an appropriate therapy or information regarding an appropriate therapy to provide to a subject. In some embodiments of any one of the methods provided herein, the determining includes providing an appropriate therapy or information regarding an appropriate therapy to a subject. As used herein, information regarding a treatment or therapy or monitoring may be provided in written form or electronic form. In some embodiments, the information may be provided as computer-readable instructions. In some embodiments, the information may be provided orally.

Anti-rejection therapies include, for example, immunosuppressives. Immunosuppressives include, but are not limited to, corticosteroids (e.g., prednisolone or hydrocortisone), glucocorticoids, cytostatics, alkylating agents (e.g., nitrogen mustards (cyclophosphamide), nitrosoureas, platinum compounds, cyclophosphamide (Cytoxan)), antimetabolites (e.g., folic acid analogues, such as methotrexate, purine analogues, such as azathioprine and mercaptopurine, pyrimidine analogues, and protein synthesis inhibitors), cytotoxic antibiotics (e.g., dactinomycin, anthracyclines, mitomycin C, bleomycin, mithramycin), antibodies (e.g., anti-CD20, anti-IL-1, anti-IL-2Ralpha, anti-T-cell or anti-CD-3 monoclonals and polyclonals, such as Atgam, and Thymoglobuline), drugs acting on immunophilins, ciclosporin, tacrolimus, sirolimus, interferons, opiods, TNF-binding proteins, mycophenolate, fingolimod and myriocin. In some embodiments, anti-rejection therapy comprises blood transfer or marrow transplant. Therapies can also include therapies for treating systemic conditions, such as sepsis. The therapy for sepsis can include intravenous fluids, antibiotics, surgical drainage, early goal directed therapy (EGDT), vasopressors, steroids, activated protein C, drotrecogin alfa (activated), oxygen and appropriate support for organ dysfunction. This may include hemodialysis in kidney failure, mechanical ventilation in pulmonary dysfunction, transfusion of blood products, and drug and fluid therapy for circulatory failure. Ensuring adequate nutrition-preferably by enteral feeding, but if necessary by parenteral nutrition-can also be included particularly during prolonged illness. Other associated therapies can include insulin and medication to prevent deep vein thrombosis and gastric ulcers.

In some embodiments, wherein infection is indicated, therapies for treating a recipient of a transplant can also include therapies for treating a bacterial, fungal and/or viral infection. Such therapies include antibiotics. Other examples include, but are not limited to, amebicides, aminoglycosides, anthelmintics, antifungals, azole antifungals, echinocandins, polyenes, diarylquinolines, hydrazide derivatives, nicotinic acid derivatives, rifamycin derivatives, streptomyces derivatives, antiviral agents, chemokine receptor antagonist, integrase strand transfer inhibitor, neuraminidase inhibitors, NNRTIs, NS5A inhibitors, nucleoside reverse transcriptase inhibitors (NRTIs), protease inhibitors, purine nucleosides, carbapenems, cephalosporins, glycylcyclines, leprostatics, lincomycin derivatives, macrolide derivatives, ketolides, macrolides, oxazolidinone antibiotics, penicillins, beta-lactamase inhibitors, quinolones, sulfonamides, and tetracyclines. Other such therapies are known to those of ordinary skill in the art. Any one of the methods provided herein can include administering or suggesting an anti-infection treatment to the subject (including providing information about the treatment to the subject, in some embodiments). In some embodiments, an anti-infection treatment may be a reduction in the amount or frequency in an immunosuppressive therapy or a change in the immunosuppressive therapy that is administered to the subject.

It has been found that particularly useful to a clinician is a report that contains the value(s) provided herein. In one aspect, therefore, such reports are provided. Reports may be in oral, written (or hard copy) or electronic form, such as in a form that can be visualized or displayed. In some embodiments, the "raw" results for each assay as provided herein are provided in a report, and from this report, further steps can be taken to analyze the amounts of total cell-free nucleic acids (such as DNA) and specific cell-free nucleic acids (such as DNA). In other embodiments, the report provides multiple values for the amounts of total cell-free nucleic acids (such as DNA) and specific cell-free nucleic acids (such as DNA). The multiple values may be from samples taken at different times, for example. In some embodiments of any one of the methods or reports provided, the total cell-free nucleic acids (such as DNA) and the specific cell-free nucleic acids (such as DNA) are provided in separate reports. In other embodiments of any one of the methods or reports provided herein, the total cell-free nucleic acids (such as DNA) and the specific cell-free nucleic acids (such as DNA) are provided in the same report.

The report(s) may be formatted to display values with respect to a threshold value and/or to include one or more threshold values. Such an arrangement or presentation may yield a quicker and easier interpretation for a clinician. For example, one or more threshold values may be indicated using lines on a graph or shading or some other indicator that allows one to be able to be informed of the threshold(s) and/or compare threshold value(s) with one or more other values on the report. In some embodiments of any one of the reports provided, the indicator may simply be any identification of one or more values as being a threshold value. In some embodiments of any one of the reports provided, the value(s) for the total cell-free nucleic acids, such as DNA, and/or the value(s) for the specific cell-free nucleic acids, such as DNA, may then be interpreted based on a comparison with the indicated threshold value(s), such as by their location relative to the threshold indicator(s), simple comparison of the values, etc. For example, if the graph is a scatter plot containing both values for total cell-free nucleic acids (such as DNA) and for specific cell-free nucleic acids (such as DNA) and the threshold values are designated with lines or the like, quadrants may be formed which may be indicative of risk in the subject. As another example, if the graph is one providing values for total cell-free nucleic acids (such as DNA) or for specific cell-free nucleic acids (such as DNA) over time and the threshold values are indicated, such as with a line or other indicator, the values of the nucleic acids in the graph may be compared with the indication of one or more threshold values. Any one of the reports provided herein or graph(s) thereof may include any one or more indicators of a threshold value.

Reports may also display a numeric output of the assay results. In this way, in some embodiments, a clinician may assess the need for a treatment for the subject or the need to monitor the subject over time.

Accordingly, in any one of the methods provided herein, the method can include assessing the amount of total cell-free nucleic acids (such as DNA) and specific cell-free nucleic acids (such as DNA) in the subject at another point in time or times. For example, the amount of total cell-free nucleic acids (such as DNA) and specific cell-free nucleic acids (such as DNA) in the subject may be assessed at one or more subsequent times, such 1, 2, 3, 4, 5, or 6 days, or 1, 2, or 3 weeks, or 1, 2, 3, 4, 5, 6, 7, 8, 9, 10, or 11 months, or one year from the earlier time point. In some embodiments of any one of the methods provided, the amount of total cell-free nucleic acids (such as DNA) and specific cell-free nucleic acids (such as DNA) in the subject may be assessed daily, biweekly, weekly, bimonthly, or monthly for a period of time, such as for 3, 6 or 9 months or 1, 2 or more years. Such assessing can be performed with any one of the methods provided herein.

Methods for determining total cell-free nucleic acids (such as DNA) as well as specific cell-free nucleic acids (such as DNA) are provided herein or are otherwise known in the art. For example, the methods of PCT Application No. PCT/US2016/030313 may be used for determining a value for the amount of specific cell-free nucleic acids (such as DNA) in a sample as provided herein. Thus, any one of the methods provided herein may include the steps of any one of the methods described in PCT Application No. PCT/US2016/030313,

Any suitable next generation or high-throughput sequencing and/or genotyping technique may be used to analyze total cell-free nucleic acids (such as DNA) as well as specific cell-free nucleic acids (such as DNA) in some embodiments. Examples of next generation and high-throughput sequencing and/or genotyping techniques include, but are not limited to, massively parallel signature sequencing, polony sequencing, 454 pyrosequencing, Illumina (Solexa) sequencing, SOLiD sequencing, ion semiconductor sequencing, DNA nanoball sequencing, heliscope single molecule sequencing, single molecule real time (SMRT) sequencing, MassARRAY®, and Digital Analysis of Selected Regions (DANSR™) (see, e.g., Stein RA (1 September 2008). "Next-Generation Sequencing Update". Genetic Engineering & Biotechnology News 28 (15); Quail, Michael; Smith, Miriam E; Coupland, Paul; Otto, Thomas D; Harris, Simon R; Connor, Thomas R; Bertoni, Anna; Swerdlow, Harold P; Gu, Yong (1 January 2012). "A tale of three next generation sequencing platforms: comparison of Ion torrent, pacific biosciences and illumina MiSeq sequencers". BMC Genomics 13 (1): 341; Liu, Lin; Li, Yinhu; Li, Siliang; Hu, Ni; He, Yimin; Pong, Ray; Lin, Danni; Lu, Lihua; Law, Maggie (1 January 2012). "Comparison of Next-Generation Sequencing Systems". Journal of Biomedicine and Biotechnology 2012: 1-11; Qualitative and quantitative genotyping using single base primer extension coupled with matrix-assisted laser desorption/ionization time-of-flight mass spectrometry (MassARRAY®). Methods Mol Biol. 2009;578:307-43; Chu T, Bunce K, Hogge WA, Peters DG. A novel approach toward the challenge of accurately quantifying fetal DNA in maternal plasma. Prenat Diagn 2010;30:1226-9; and Suzuki N, Kamataki A, Yamaki J, Homma Y. Characterization of circulating DNA in healthy human plasma. Clinica chimica acta; International Journal of Clinical Chemistry 2008;387:55-8).

Likewise, the methods of measuring cell-free DNA of U.S. Publication No. 2015-0086477-A1 can be included as part of any one of the methods provided herein for determining a value for the amount of cell-free nucleic acids (such as DNA).

In some embodiments of any one of the methods provided herein the PCR is quantitative PCR, meaning that amounts of nucleic acids can be determined. Quantitative PCR include real-time PCR, digital PCR, TAQMAN™, etc. In some embodiments of any one of the methods provided herein the PCR is "real-time PCR". Such PCR refers to a PCR reaction where the reaction kinetics can be monitored in the liquid phase while the amplification process is still proceeding. In contrast to conventional PCR, real-time PCR offers the ability to simultaneously detect or quantify in an amplification reaction in real time. Based on the increase of the fluorescence intensity from a specific dye, the concentration of the target can be determined even before the amplification reaches its plateau.

The use of multiple probes can expand the capability of single-probe real-time PCR. Multiplex real-time PCR uses multiple probe-based assays, in which each assay can have a specific probe labeled with a unique fluorescent dye, resulting in different observed colors for each assay. Real-time PCR instruments can discriminate between the fluorescence generated from different dyes. Different probes can be labeled with different dyes that each have unique emission spectra. Spectral signals are collected with discrete optics, passed through a series of filter sets, and collected by an array of detectors. Spectral overlap between dyes may be corrected by using pure dye spectra to deconvolute the experimental data by matrix algebra. Other methods would be apparent to those of ordinary skill in the art.

As mentioned above, in some embodiments, any one of the methods provided herein may include steps of a "mismatch amplification method" or "mismatch amplification-based quantitative assay" or the like in order to determine a value for an amount of specific cell-free nucleic acids (such as DNA). In some embodiments of any one of the methods provided herein, the mismatch amplification-based quantitative assay is any quantitative assay whereby nucleic acids are amplified with the MOMA primers as described herein, and the amounts of the nucleic acids can be determined. Such methods comprise multiple amplifications from multiple SNV targets. Such methods include the methods of PCT Application No. PCT/US2016/030313, and any one of the methods provided herein may include the steps of any one of the methods described in PCT Application No. PCT/US2016/030313 In some embodiments of any one of the methods provided herein, such results of the multiple amplifications may be used to determine an amount of non-native nucleic acids in a sample by using one or more statistical methods, including the median, robust standard deviation, robust coefficient of variation, and discordance value. In some embodiments of any one of the methods provided herein, the mismatch amplification-based quantitative assay is any quantitative assay whereby nucleic acids are amplified with the MOMA primers as described herein, and the amounts of the nucleic acids can be determined.

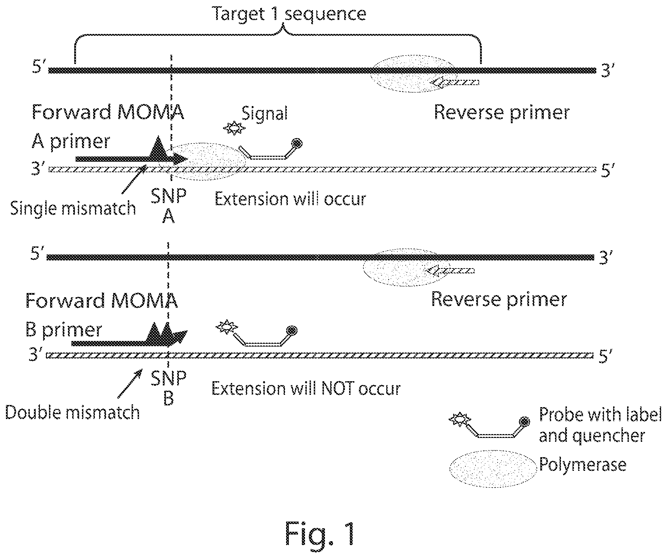

Primers for use in such assays may be obtained, and any one of the methods provided herein can include a step of obtaining one or more primer pairs for performing the quantitative assays. Generally, the primers possess unique properties that facilitate their use in quantifying amounts of nucleic acids. For example, a forward primer of a primer pair can be mismatched at a 3' nucleotide (e.g., penultimate 3' nucleotide). In some embodiments of any one of the methods or compositions provided, this mismatch is at a 3' nucleotide but adjacent to the SNV position. In some embodiments of any one of the methods or composition provided, the mismatch positioning of the primer relative to a SNV position is as shown in **Fig. 1.** Generally, such a forward primer even with the 3' mismatch to produce an amplification product (in conjunction with a suitable reverse primer) in an amplification reaction, thus allowing for the amplification and resulting detection of a nucleic acid with the respective SNV. If the particular SNV is not present, and there is a double mismatch with respect to the other allele of the SNV target, an amplification product will generally not be produced. Preferably, in some embodiments of any one of the methods provided herein, for each SNV target a primer pair is obtained whereby specific amplification of each allele can occur without amplification of the other allele(s). "Specific amplification" refers to the amplification of a specific allele of a target without substantial amplification of another nucleic acid or without amplification of another nucleic acid sequence above background or noise. In some embodiments, specific amplification results only in the amplification of the specific allele.

As used herein, "single nucleotide variant" refers to a nucleic acid sequence within which there is sequence variability at a single nucleotide. In some embodiments, the SNV is a biallelic SNV, meaning that there is one major allele and one minor allele for the SNV. In some embodiments, the SNV may have more than two alleles, such as within a population. Generally, a "minor allele" refers to an allele that is less frequent in a set of nucleic acids, for a locus, while a "major allele" refers to the more frequent allele in a set of nucleic acids. The methods provided herein can quantify nucleic acids of major and minor alleles within a mixture of nucleic acids even when present at low levels, in some embodiments.

The nucleic acid sequence within which there is sequence identity variability, such as a SNV, is generally referred to as a "target". As used herein, a "SNV target" refers to a nucleic acid sequence within which there is sequence variability at a single nucleotide, such as in a population of individuals. The SNV target has more than one allele, and in preferred embodiments, the SNV target is biallelic. In some embodiments of any one of the methods provided herein, the SNV target is a SNP target. In some of these embodiments, the SNP target is biallelic. It has been discovered that nucleic acids can be quantified even at extremely low levels by performing amplification-based quantitative assays, such as PCR assays with primers specific for SNV targets. In some embodiments, the amount of nucleic acids is determined by attempting amplification-based quantitative assays, such as quantitative PCR assays, with primers for a plurality of SNV targets. A "plurality of SNV targets" refers to more than one SNV target where for each target there are at least two alleles. Preferably, in some embodiments, each SNV target is expected to be biallelic and a primer pair specific to each allele of the SNV target is used to specifically amplify nucleic acids of each allele, where amplification occurs if the nucleic acid of the specific allele is present in the sample.

In some embodiments of any one of the methods provided herein, for each SNV target that is biallelic, there are two primer pairs, each specific to one of the two alleles and thus have a single mismatch with respect to the allele it is to amplify and a double mismatch with respect to the allele it is not to amplify (again if nucleic acids of these alleles are present). In some embodiments of any one of the methods provided herein, the mismatch primer is the forward primer. In some embodiments of any one of the methods provided herein, the reverse primer of the two primer pairs for each SNV target is the same.

These concepts can be used in the design of primer pairs for any one of the methods provided herein. It should be appreciated that the forward and reverse primers are designed to bind opposite strands (e.g., a sense strand and an antisense strand) in order to amplify a fragment of a specific locus of the template. The forward and reverse primers of a primer pair may be designed to amplify a nucleic acid fragment of any suitable size to detect the presence of, for example, an allele of a SNV target according to the disclosure. Any one of the methods provided herein can include one or more steps for obtaining one or more primer pairs as described herein.

In some embodiments, the amplification-based quantitative assay, such as quantitative PCR, is performed with primer pairs for at least 40, 45, 50, 55, 60, 65, 70, 75, 80, 85, 90, 91, 92, 93, 94, 95 or more targets. In some embodiments, the quantitative assay is performed with primer pairs for fewer than 105, 104, 103, 102, 101,100, 99, 98 or 97 targets. In some embodiments, sufficient informative results are obtained with primer pairs for between 40-105, 45-105, 50-105, 55-105, 60-105, 65-105, 70-105, 75-105, 80-105, 85-105, 90-105, 90-104, 90-103, 90-102, 90-101, 90-100, 90-99, 91-99, 92-99, 93, 99, 94-99, 95-99, or 90-95 targets. In some embodiments, sufficient informative results are obtained with primer pairs for between 40-99, 45-99, 50-99, 55-99, 60-99, 65-99, 70-99, 75-99, 80-99, 85-99, 90-99, 90-99, 90-98, 90-97 or 90-96 targets. In still other embodiments, sufficient informative results are obtained with primer pairs for between 40-95, 45-95, 50-95, 55-95, 60-95, 65-95, 70-95, 75-95, 80-95, 85-95, or 90-95 targets. In still other embodiments, sufficient informative results are obtained with primer pairs for between 40-90, 45-90, 50-90, 55-90, 60-90, 65-90, 70-90, 75-90, 80-90, or 85-90. In still other embodiments, sufficient informative results are obtained with primer pairs for between 40-85, 45-85, 50-85, 55-85, 60-85, 65-85, 70-85, 75-85, or 80-85. In still other embodiments, sufficient informative results are obtained with primer pairs for between 40-80, 45-80, 50-80, 55-80, 60-80, 65-80, 70-80, or 75-80. In still other embodiments, sufficient informative results are obtained with primer pairs for between 40-75, 45-75, 50-75, 55-75, 60-75, 65-75, or 70-75 targets.

Generally, "informative results" as provided herein are the results that can be used to quantify the level of specific-native or non-specific nucleic acids in a sample. Informative results can exclude the results where the native nucleic acids are heterozygous for a specific SNV target as well as "no call" or erroneous call results. In some embodiments of any one of the methods provided, the amount of specific-native and/or non-specific nucleic acids represents an average across informative results for the nucleic acids, respectively. In some embodiments of any one of the methods provided herein, this average is given as an absolute amount or as a percentage. Preferably, in some embodiments of any one of the methods provided herein, this average is the median. In other embodiments of any one of the methods provided herein, the average is a trimmed mean. As used herein, the "trimmed mean" refers to the removal of the lowest reporting targets (such as the two lowest) in combination with the highest of the reporting targets (such as the two highest). In still other embodiments of any one of the methods provided herein, the average is the mean.

In some embodiments of any one of the methods provided herein, the method can further comprise the use of Robust Statistics (e.g., BD FACSDiva™ Software) to analyze the results. In some of such embodiments, the use of such statistics can be done at the end as a quality check of the results. In some of such embodiments, the statistics may indicate a sample may need to be rerun or some results should be discarded. In some embodiments, any one of the methods provided herein can include a step whereby a Standard Deviation, such as a Robust Standard Deviation (rSD), and/or a Coefficient of Variation, such as a Robust Coefficient of Variation (rCV), or % Coefficient of Variation, such as a % Robust Coefficient of Variation, can be calculated.

As used herein, the Robust SD is based upon the deviation of individual data points to the median of the population. It can be calculated as:

$$rSD = (Median\ of\{|X_i - Median_x|\}) \times 1.4826$$

The value 1.4826 is a constant factor that adjusts the resulting robust value to the equivalent of a normal population distribution. Thus, for a normally distributed population, the SD and the rSD are equal.

Similarly, the Robust CV and percent Robust CV can be calculated as:

$$\mathbf{rCV = rSD/Median_x}\ \text{and}\ \mathbf{\%\ rCV = rSD/Median_x \times 100\%},$$

respectively.

Thus, in any one of the methods provided herein the final amounts can be determined at least in part on an analysis of the results using a Standard Deviation, such as rSD, and/or a Coefficient of Variation, such as rCV, or % Coefficient of Variation, such as %rCV.

In some embodiments of any one of the methods provided herein, the method can further comprise the use of a discordance value (dQC). For example, the average minor allele proportion of recipient homozygous and non-informative targets can be evaluated in order to safeguard against sample mixups and contamination. These should theoretically read nearly zero percent, subject to non-specificity allelic noise. If a sample-swap had occurred during collection or processing, the wrong recipient genotypes are used, the dQC immediately flags up to 50 or 100% readings at presumed non-informative targets. The dQC can also captures sample contamination and possibly genomic instability. Generally, healthy samples will have a dQC below 0.5%.

The amount, such as ratio or percentage, of specific nucleic acids may be determined with the quantities of the major and minor alleles as well as genotype, as needed. In some embodiments of any one of the methods provided herein, the alleles can be determined based on prior genotyping (e.g., of the recipient and/or donor, respectively). Methods for genotyping are well known in the art. Such methods include sequencing, such as next generation, hybridization, microarray, other separation technologies or PCR assays. Any one of the methods provided herein can include steps of obtaining such genotypes.

It should be appreciated that the primer pairs described herein may be used in a multiplex PCR assay. Accordingly, in some embodiments, the primer pairs are designed to be compatible with other primer pairs in a PCR reaction. For example, the primer pairs may be designed to be compatible with at least 2, at least 5, at least 10, at least 20, at least 30, at least 40, etc. other primer pairs in a PCR reaction. As used herein, primer pairs in a PCR reaction are "compatible" if they are capable of amplifying their target in the same PCR reaction. In some embodiments, primer pairs are compatible if the primer pairs are inhibited from amplifying their target nucleic acid (such as DNA) by no more than 1%, no more than 2%, no more than 5%, no more than 10%, no more than 15%, no more than 20%, no more than 25%, no more than 30%, no more than 35%, no more than 40%, no more than 45%, no more than 50%, or no more than 60% when multiplexed in the same PCR reaction. Primer pairs may not be compatible for a number of reasons including, but not limited to, the formation of primer dimers and binding to off-target sites on a template that may interfere with another primer pair. Accordingly, the primer pairs of the disclosure may be designed to prevent the formation of dimers with other primer pairs or limit the number of off-target binding sites. Exemplary methods for designing primers for use in a multiplex PCR assay are known in the art and are otherwise described herein.

In some embodiments of any one of the methods provided herein, the quantitative assays include quantitative PCR assays. Quantitative PCR include real-time PCR, digital PCR, TAQMAN™, etc. In some embodiments of any one of the methods provided herein the PCR is "Real-time PCR". Such PCR refers to a PCR reaction where the reaction kinetics can be monitored in the liquid phase while the amplification process is still proceeding. In contrast to conventional PCR, real-time PCR offers the ability to simultaneously detect or quantify in an amplification reaction in real time. Based on the increase of the fluorescence intensity from a specific dye, the concentration of the target can be determined even before the amplification reaches its plateau.

The use of multiple probes can expand the capability of single-probe real-time PCR. Multiplex real-time PCR uses multiple probe-based assays, in which each assay can have a specific probe labeled with a unique fluorescent dye, resulting in different observed colors for each assay. Real-time PCR instruments can discriminate between the fluorescence generated from different dyes. Different probes can be labeled with different dyes that each have unique emission spectra. Spectral signals can be collected with discrete optics, passed through a series of filter sets, and collected by an array of detectors. Spectral overlap between dyes may be corrected by using pure dye spectra to deconvolute the experimental data by matrix algebra.

In any one of the methods provided herein the PCR may be digital PCR. Digital PCR involves partitioning of diluted amplification products into a plurality of discrete test sites such that most of the discrete test sites comprise either zero or one amplification product. The amplification products are then analyzed to provide a representation of the frequency of the selected genomic regions of interest in a sample. Analysis of one amplification product per discrete test site results in a binary "yes-or-no" result for each discrete test site, allowing the selected genomic regions of interest to be quantified and the relative frequency of the selected genomic regions of interest in relation to one another be determined. In certain aspects, in addition to or as an alternative, multiple analyses may be performed using amplification products corresponding to genomic regions from predetermined regions. Results from the analysis of two or more predetermined regions can be used to quantify and determine the relative frequency of the number of amplification products. Using two or more predetermined regions to determine the frequency in a sample reduces a possibility of bias through, e.g., variations in amplification efficiency, which may not be readily apparent through a single detection assay. Methods for quantifying DNA using digital PCR are known in the art and have been previously described, for example in U.S. Patent Publication number US 2014-0242582.

Any one of the methods provided herein can comprise extracting nucleic acids, such as cell-free DNA, from a sample obtained from a subject, such as a recipient of a transplant. Such extraction can be done using any method known in the art or as otherwise provided herein (see, e.g., Current Protocols in Molecular Biology, latest edition, or the QIAamp circulating nucleic acid kit, or other appropriate commercially available kits). An exemplary method for isolating cell-free DNA from blood is described. Blood containing an anticoagulant such as EDTA or DTA is collected from a subject. The plasma, which contains cf-DNA, is separated from cells present in the blood (e.g., by centrifugation or filtering). An optional secondary separation may be performed to remove any remaining cells from the plasma (e.g., a second centrifugation or filtering step). The cf-DNA can then be extracted using any method known in the art, e.g., using a commercial kit such as those produced by Qiagen. Other exemplary methods for extracting cf-DNA are also known in the art (see, e.g., Cell-Free Plasma DNA as a Predictor of Outcome in Severe Sepsis and Septic Shock. Clin. Chem. 2008, v. 54, p. 1000-1007; Prediction of MYCN Amplification in Neuroblastoma Using Serum DNA and Real-Time Quantitative Polymerase Chain Reaction. JCO 2005, v. 23, p.5205-5210; Circulating Nucleic Acids in Blood of Healthy Male and Female Donors. Clin. Chem. 2005, v. 51, p.1317-1319; Use of Magnetic Beads for Plasma Cell-free DNA Extraction: Toward Automation of Plasma DNA Analysis for Molecular Diagnostics. Clin. Chem. 2003, v. 49, p. 1953-1955; Chiu RWK, Poon LLM, Lau TK, Leung TN, Wong EMC, Lo YMD. Effects of blood-processing protocols on fetal and total DNA quantification in maternal plasma. Clin Chem 2001;47:1607-1613; and Swinkels et al. Effects of Blood-Processing Protocols on Cell-free DNA Quantification in Plasma. Clinical Chemistry, 2003, vol. 49, no. 3, 525-526).

In some embodiments of any one of the methods provided herein, an early additional amplification step is performed. An exemplary method of amplification is as follows, and such a method can be included in any one of the methods provided herein. ~15 ng of cell free plasma DNA is amplified in a PCR using Q5 DNA polymerase with approximately ~100 targets where pooled primers were at 6uM total. Samples undergo approximately 35 cycles. Reactions are in 25 ul total. After amplification, samples can be cleaned up using several approaches including AMPURE bead cleanup, bead purification, or simply Exosap it, or Zymo.

Various aspects of the present invention may be used alone, in combination, or in a variety of arrangements not specifically discussed in the embodiments described in the foregoing and are therefore not limited in their application to the details and arrangement of components set forth in the foregoing description or illustrated in the drawings. For example, aspects described in one embodiment may be combined in any manner with aspects described in other embodiments.

Also, embodiments of the invention may be implemented as one or more methods, of which an example has been provided. The acts performed as part of the method(s) may be ordered in any suitable way. Accordingly, embodiments may be constructed in which acts are performed in an order different from illustrated, which may include performing some acts simultaneously, even though shown as sequential acts in illustrative embodiments.

Use of ordinal terms such as "first," "second," "third," etc., in the claims to modify a claim element does not by itself connote any priority, precedence, or order of one claim element over another or the temporal order in which acts of a method are performed. Such terms are used merely as labels to distinguish one claim element having a certain name from another element having a same name (but for use of the ordinal term).

The phraseology and terminology used herein is for the purpose of description and should not be regarded as limiting. The use of "including," "comprising," "having," "containing", "involving", and variations thereof, is meant to encompass the items listed thereafter and additional items.

Having described several embodiments of the invention in detail, various modifications and improvements will readily occur to those skilled in the art. Accordingly, the foregoing description is by way of example only, and is not intended as limiting. The following description provides examples of the methods provided herein.

## EXAMPLES

### Example 1 - Mismatch Amplification Assay (MOMA)

*SNV Target Selection*

Identification of targets for multiplexing in accordance with the disclosure may include one or more of the following steps, as presently described. First, highly heterozygous SNPs can be screened on several ethnic control populations (Hardy-Weinberg p > 0.25), excluding known difficult regions. Difficult regions include syndromic regions likely to be abnormal in patients and regions of low complexity, including centromeres and telomeres of chromosomes. Target fragments of desired lengths can then be designed *in silico.* Specifically, two 20-26 bp primers spanning each SNP's 70 bp window can be designed. All candidate primers can then be queried to GCRh37 using BLAST. Those primers that were found to be sufficiently specific can be retained, and monitored for off-target hits, particularly at the 3' end of the fragment. The off-target candidate hits can be analyzed for pairwise fragment generation that would survive size selection. Selected primers can then be subjected to an *in silico* multiplexing evaluation. The primers' computed melting temperatures and guanine-cytosine percentages (GC%) can be used to filter for moderate range sequences. An iterated genetic algorithm and simulated annealing can be used to select candidate primers compatible for 400 targets, ultimately resulting in the selection of 800 primers. The 800 primers can be generated and physically tested for multiplex capabilities at a common melting temperature in a common solution. Specifically, primers can be filtered based on even amplification in the multiplex screen and moderate read depth window. Forty-eight assays can be designed for MOMA using the top performing multiplexed SNPs. Each SNP can have a probe designed in WT/MUT at four mismatch choices; eight probes per assay. The new nested primers can be designed within the 70 bp enriched fragments. Finally, the primers can be experimentally amplified to evaluate amplification efficiency (8 probes x 48 assays in triplicate, using TAQMAN™).

*A priori Genotyping Informativeness of each Assay*

Using, for example, known or possible native and non-native genotypes at each assayed SNP, a subset of informative assays was selected. Note that subject homozygous sites can be used where the non-native is any other genotype. Additionally, if the non-native genotype is not known, it can be inferred. Genotypes may also be learned through sequencing, SNP microarray, or application of a MOMA assay on known 0% (clean recipient) samples.

*Post Processing Analysis of Multiplex Assay Performance*

Patient-specific MOMA probe biases can be estimated across an experimental cohort. Selection iteratively can be refined to make the final non-native percent call.

*Reconstruction Experiment*

The sensitivity and precision of the assay can be evaluated using reconstructed plasma samples with known mixing ratios. Specifically, the ratios of 1:10, 1:20, 1:100, 1:200, and 1:1000 can be evaluated. Generally, primers for 95 SNV targets can be used as described herein in some embodiments.

To work without non-native genotype information, the following procedure may be performed to infer informative assays and allow for quantification of non-native-specific cell-free DNA in plasma samples. All assays can be evaluated for performance in the full information scenario. This procedure thus assumed clean AA/AB/BB genotypes at each assay and unbiased behavior of each quantification. With native genotype, assays known to be homozygous in the subject can be selected. Contamination can be attributed to the non-native nucleic acids, and the assay collection created a tri-modal distribution with three clusters of assays corresponding to the non-, half-, and fully-informative assays. With sufficient numbers of recipient homozygous assays, the presence of non-native fully informative assays can be assumed.

If the native genotype is homozygous and known, then if a measurement that is not the non-native genotype is observed, the probes which are truly non-native-homozygous will have the highest cluster and equal the guess whereas those that are non-native heterozygous will be at half the guess. A probability distribution can be plotted and an expectation maximization algorithm (EM) can be employed to infer non-native genotype. Such can be used to infer the non-native genotype frequency in any one of the methods provided herein.

Accordingly, an EM algorithm was used to infer the most likely non-native genotypes at all assayed SNV targets. With inferred non-native genotypes, quantification may proceed as in the full-information scenario. EM can begin with the assumption that the minor allele ratio found at an assay follows a tri-modal distribution, one for each combination of subject and non-native, given all assays are "AA" in the subject (or flipped from "BB" without loss of generality). With all non-native genotypes unknown, it is possible to bootstrap from the knowledge that any assays exhibiting nearly zero minor allele are non-native AA, and the highest is non-native BB. Initial guesses for all non-native genotypes were recorded, and the mean of each cluster calculated. Enforcing that the non-native BB assays' mean is twice that of the non-native AB restricts the search. The algorithm then reassigns guessed non-native genotypes based on the clusters and built-in assumptions. The process was iterative until no more changes were made. The final result is a set of the most likely non-native genotypes given their measured divergence from the background. Generally, every target falls into the model; a result may be tossed if between groups after maximization.

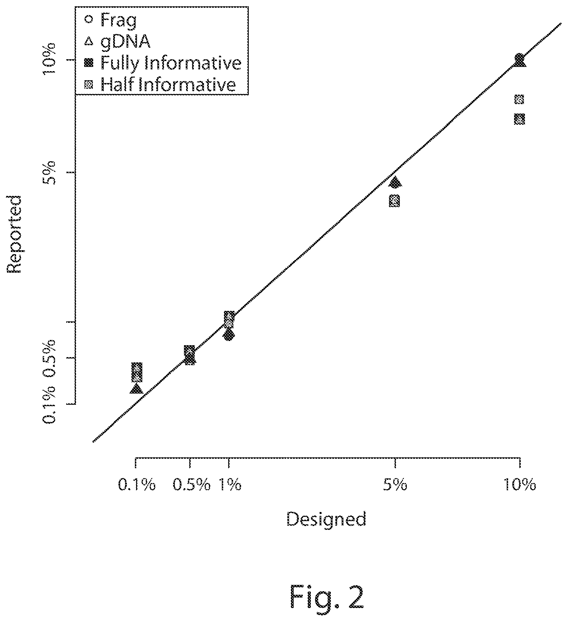

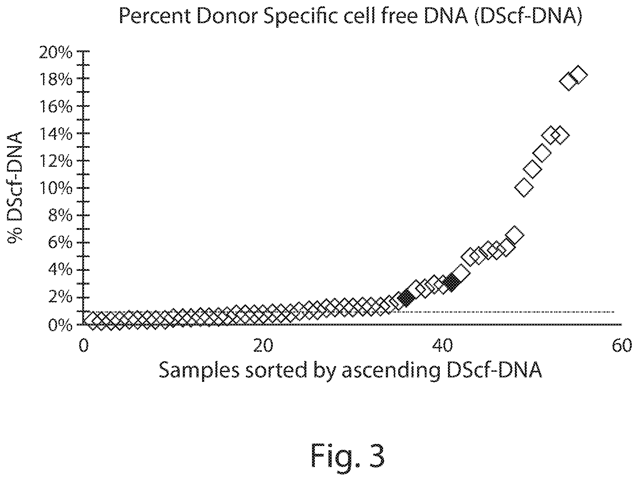

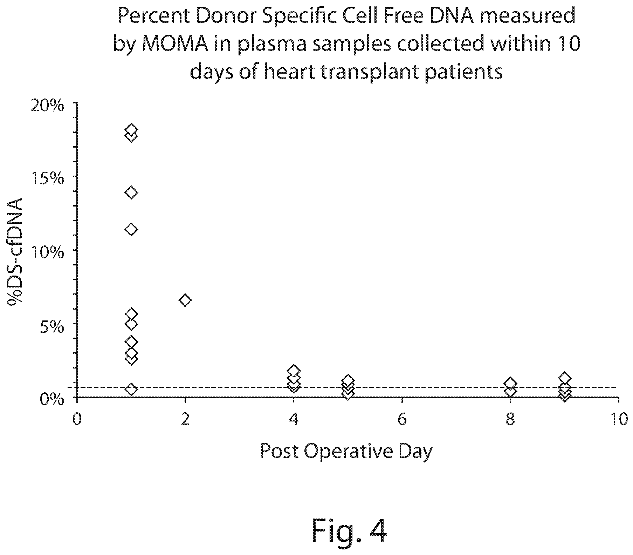

Results of the reconstruction experiment demonstrate proof of concept **(Fig. 2).** One target is fully informative where there is a homozygous donor against a homozygous recipient (shaded data points). The other target is half informative where there is a heterozygous donor against a homozygous recipient (open data points). In addition, plasma samples from transplant recipient patients were analyzed with a mismatch method as described herein **(Fig. 3).** All data comes from patients who have had biopsies. Dark points denote rejection. Further data shown in **Fig. 4,** demonstrate that a mismatch method as provided herein worked with real plasma samples. After transplant surgery, the donor percent levels dropped off. Primers for 95 SNV targets as described herein were used.

## Example 2 - Exemplary Assays

*Genotyping*

A multiplexed, allele-specific quantitative PCR-based assay can be used to calculate donor fraction (DF) as a percentage of cf-DNA. A panel of high frequency SNPs are selected for their ability to reliably discriminate between alleles. Briefly, 15 ng of total cf-DNA is added to a multiplexed library master mixture with an exogenous standard spiked into each sample (4.5E+03 copies) and amplified by PCR for 35 cycles in a 25 ul reaction containing 0.005 U Q5 (NEB) DNA polymerase, 0.2 mM dNTPs, 3 uM forward primer pool of 96 targets, 3 uM reverse primer pool of 96 targets, at a final concentration of 2 mM MgCl2.

Cycling conditions can be 98°C for 30s, then 35 cycles of 98°C for 10s, 55°C for 40s, and 72°C for 30s. This can then be finished with a 2-minute incubation at 72°C and then stored at 4°C. Ten microliters of the final reaction is cleaned up with ExoSAP-IT (Thermo Fisher Scientific) by incubating at 37°C for 15 minutes followed by 80°C for 15 minutes. Libraries are then diluted with Preservation Buffer and either processed for genotyping or stored at -80°C. Quantitative genotyping (qGT) is performed starting from 3 8 ul of a 1:100 dilution of the preserved library diluted 1:100 and run in duplicate 3 ul reactions with appropriate controls and calibrators on the Roche LightCycler 480 platform (Roche Diagnostics, Indianapolis, IN). A procedure is used to assign the genomic DNA (gDNA) of the recipient or donor with one of three possible genotypes at each target loci (i.e. homozygous AA, heterozygous AB and homozygous BB).

*Donor fraction (specific) analysis*

Standard curves of heterozygous DNA sources are used to quantify alleles at each target. Quality control procedures can be used to evaluate each standard curve and sample amplification. Quantifiable targets can proceed to interpretation. Acceptability criteria can include historic amplification shape, specificity of the allele specific PCR assay with respect to the second allele, signal to noise, slope and r-squared of standard curve sets, amplification of controls, and contamination of negative controls.

With the labels of recipient and/or donor possible genotypes at each target (e.g. homozygous AA, heterozygous AB, and homozygous BB, informative targets can be defined as those where the recipient is known homozygous and the donor has a different genotype. Where the donor is homozygous and different from the recipient the target is referred to as fully-informative, because the observed B allele ratio is approximately the overall DF level. Where the donor is heterozygous the target is called half-informative because the contribution is to both the A and B alleles, and the measured contribution is doubled. The median of informative and quality-control-passed allele ratios is calculated and reported as DF (%) of total cf-DNA.

Each quantitative genotyping process can yield two quality control measures, the rCV and dQC. The regularized robust coefficient of variation (rCV) is computed using the distribution of the informative and quantifiable targets. First the robust standard deviation (rSD) is computed as the median absolute divergence from the median minor species proportion. The rSD is converted to a coefficient of variation by dividing by the median after it has been regularized. The rCV measures the spread of assayed targets around their median and can serve as a metric of precision or sample quality. The dQC is a discordance quality check, such as an evaluation of the average minor allele proportion of recipient homozygous and non-informative targets (can be performed as a safeguard against contamination.)

### Example 3 - Determination of Total Cell-free DNA

In some embodiments, the total cell-free DNA (cf-DNA) is determined. Three to ten milliliters (ml) of anti-coagulated blood is collected in 10 ml Cell-Free DNA Blood Collection Tubes (BCT) tubes (Streck, Omaha, NE). Plasma is separated from whole blood by centrifugation and stored at -80°C until DNA extraction. Cf-DNA extractions may be performed using ReliaPrep™ HT Circulating Nucleic Acid Kit, Custom (Promega, Madison, WI).

Total cf-DNA content from plasma is evaluated in triplicate by quantitative real-time PCR as previously described (Hidestrand et al.. Influence of temperature during transportation on cell-free DNA analysis. Fetal Diagn Ther 31, 122-128 (2012)). PCR analysis is carried out on an Applied Biosystems QuantStudio 7 Flex Real-Time PCR System (Thermo Fisher Scientific, Waltham, MA).

### Example 4 - Assessing Risk using Total and Specific Cell-free DNA

As shown in **Fig. 7,** the values for total cell-free nucleic acids (such as DNA) and values for specific cell-free nucleic acids (such as DNA) can be displayed together for various time points in a report.

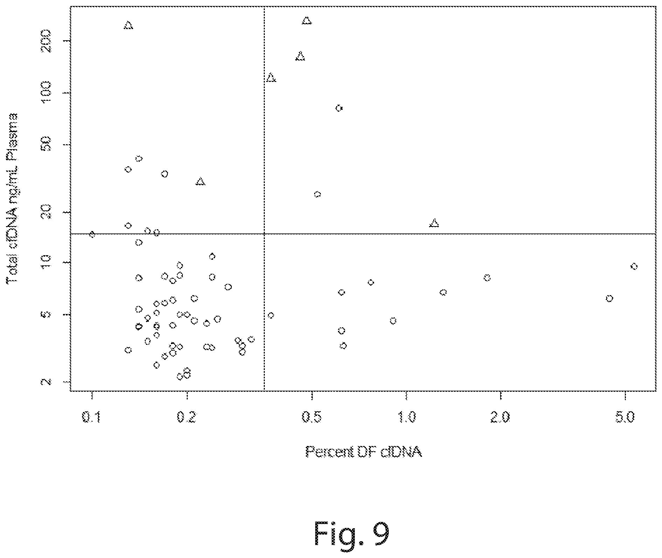

**Fig. 9** shows another example of a report showing both types of data. Data from 68 patients was analyzed. As expected, when both the values for the total cell-free DNA and specific cell-free DNA are considered high, rejection is likely occurring and driving an inflammatory process (upper right quadrant) or an adverse outcome or prognosis, such as death, is indicated. When the total cell-free DNA is considered high but specific cell-free DNA is not, there is likely to be an infection occurring in the subject but not rejection (upper left quadrant). However, when the total cell-free DNA is considered low along with what is considered high specific cell-free DNA, it is likely the subject is in an early stage of rejection and would otherwise be considered asymptomatic (lower right quadrant). Anti-rejection treatment and/or further monitoring could still be needed. Finally, when both total cell-free DNA and specific cell-free DNA are considered low, it is expected that no treatment is warranted, although it may still be necessary to continue monitoring the patient (lower left quadrant).

### Example 5 - Examples of Computer-Implemented Embodiments

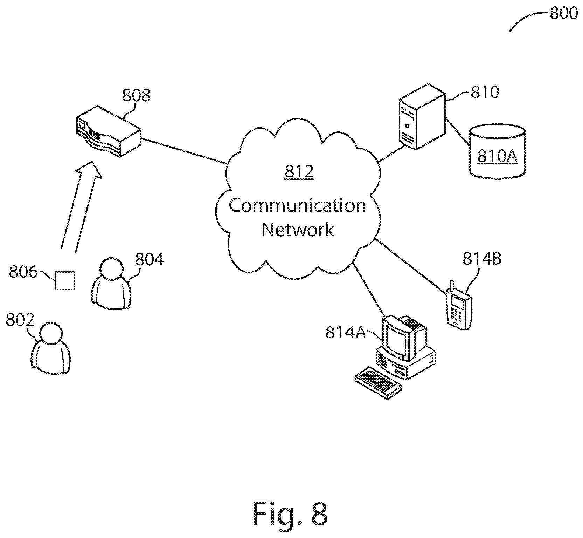

In some embodiments, the diagnostic techniques described above may be implemented via one or more computing devices executing one or more software facilities to analyze samples for a subject over time, measure cell-free nucleic acids (such as DNA) in the samples, and produce a diagnostic result based on one or more of the samples. **Fig. 8** illustrates an example of a computer system with which some embodiments may operate, though it should be appreciated that embodiments are not limited to operating with a system of the type illustrated in **Fig. 8.**

The computer system of **Fig. 8** includes a subject 802 and a clinician 804 that may obtain a sample 806 from the subject 806. As should be appreciated from the foregoing, the sample 806 may be any suitable sample of biological material for the subject 802 that may be used to measure the presence of cell-free nucleic acids (such as DNA) in the subject 802, including a blood sample. The sample 806 may be provided to an analysis device 808, which one of ordinary skill will appreciate from the foregoing will analyze the sample 808 so as to determine (including estimate) a total amount of cell-free nucleic acids (such as DNA) and an amount of a specific cell-free nucleic acids (such as DNA) in the sample 806 and/or the subject 802. For ease of illustration, the analysis device 808 is depicted as single device, but it should be appreciated that analysis device 808 may take any suitable form and may, in some embodiments, be implemented as multiple devices. To determine the amounts of cell-free nucleic acids (such as DNA) in the sample 806 and/or subject 802, the analysis device 808 may perform any of the techniques described above, and is not limited to performing any particular analysis. The analysis device 808 may include one or more processors to execute an analysis facility implemented in software, which may drive the processor(s) to operate other hardware and receive the results of tasks performed by the other hardware to determine on overall result of the analysis, which may be the amounts of cell-free nucleic acids (such as DNA) in the sample 806 and/or the subject 802. The analysis facility may be stored in one or more computer-readable storage media, such as a memory of the device 808. In other embodiments, techniques described herein for analyzing a sample may be partially or entirely implemented in one or more special-purpose computer components such as Application Specific Integrated Circuits (ASICs), or through any other suitable form of computer component that may take the place of a software implementation.

In some embodiments, the clinician 804 may directly provide the sample 806 to the analysis device 808 and may operate the device 808 in addition to obtaining the sample 806 from the subject 802, while in other embodiments the device 808 may be located geographically remote from the clinician 804 and subject 802 and the sample 806 may need to be shipped or otherwise transferred to a location of the analysis device 808. The sample 806 may in some embodiments be provided to the analysis device 808 together with (e.g., input via any suitable interface) an identifier for the sample 806 and/or the subject 802, for a date and/or time at which the sample 806 was obtained, or other information describing or identifying the sample 806.

The analysis device 808 may in some embodiments be configured to provide a result of the analysis performed on the sample 806 to a computing device 810, which may include a data store 810A that may be implemented as a database or other suitable data store. The computing device 810 may in some embodiments be implemented as one or more servers, including as one or more physical and/or virtual machines of a distributed computing platform such as a cloud service provider. In other embodiments, the device 810 may be implemented as a desktop or laptop personal computer, a smart mobile phone, a tablet computer, a special-purpose hardware device, or other computing device.

In some embodiments, the analysis device 808 may communicate the result of its analysis to the device 810 via one or more wired and/or wireless, local and/or wide-area computer communication networks, including the Internet. The result of the analysis may be communicated using any suitable protocol and may be communicated together with the information describing or identifying the sample 806, such as an identifier for the sample 806 and/or subject 802 or a date and/or time the sample 806 was obtained.

The computing device 810 may include one or more processors to execute a diagnostic facility implemented in software, which may drive the processor(s) to perform diagnostic techniques described herein. The diagnostic facility may be stored in one or more computer-readable storage media, such as a memory of the device 810. In other embodiments, techniques described herein for analyzing a sample may be partially or entirely implemented in one or more special-purpose computer components such as Application Specific Integrated Circuits (ASICs), or through any other suitable form of computer component that may take the place of a software implementation.

The diagnostic facility may receive the result of the analysis and the information describing or identifying the sample 806 and may store that information in the data store 810A. The information may be stored in the data store 810A in association with other information for the subject 802, such as in a case that information regarding prior samples for the subject 802 was previously received and stored by the diagnostic facility. The information regarding multiple samples may be associated using a common identifier, such as an identifier for the subject 802. In some cases, the data store 810A may include information for multiple different subjects.

The diagnostic facility may also be operated to analyze results of the analysis of one or more samples 806 for a particular subject 802, identified by user input, so as to determine a diagnosis for the subject 802. The diagnosis may be a conclusion of a risk that the subject 802 has, may have, or may in the future develop a particular condition. The diagnostic facility may determine the diagnosis using any of the various examples described above, including by comparing the amounts of cell-free nucleic acids (such as DNA) determined for a particular sample 806 to one or more thresholds or by comparing a change over time in the amounts of cell-free nucleic acids (such as DNA) determined for samples 806 over time to one or more thresholds. For example, the diagnostic facility may determine a risk to the subject 802 of a condition by comparing a total amount of cell-free nucleic acids (such as DNA) for one or more samples 806 to one threshold and comparing an amount of a specific cell-free nucleic acids (such as DNA) for the same sample(s) 806 to another threshold. Based on the comparisons to the thresholds, the diagnostic facility may produce an output indicative of a risk to the subject 802 of a condition.

As should be appreciated from the foregoing, in some embodiments, the diagnostic facility may be configured with different thresholds to which amounts of cell-free nucleic acids (such as DNA) may be compared. The different thresholds may, for example, correspond to different demographic groups (age, gender, race, economic class, presence or absence of a particular procedure/condition/other in medical history, or other demographic categories), different conditions, and/or other parameters or combinations of parameters. In such embodiments, the diagnostic facility may be configured to select thresholds against which amounts of cell-free nucleic acids (such as DNA) are to be compared, with different thresholds stored in memory of the computing device 810. The selection may thus be based on demographic information for the subject 802 in embodiments in which thresholds differ based on demographic group, and in these cases demographic information for the subject 802 may be provided to the diagnostic facility or retrieved (from another computing device, or a data store that may be the same or different from the data store 810A, or from any other suitable source) by the diagnostic facility using an identifier for the subject 802. The selection may additionally or alternatively be based on the condition for which a risk is to be determined, and the diagnostic facility may prior to determining the risk receive as input a condition and use the condition to select the thresholds on which to base the determination of risk. It should be appreciated that the diagnostic facility is not limited to selecting thresholds in any particular manner, in embodiments in which multiple thresholds are supported.

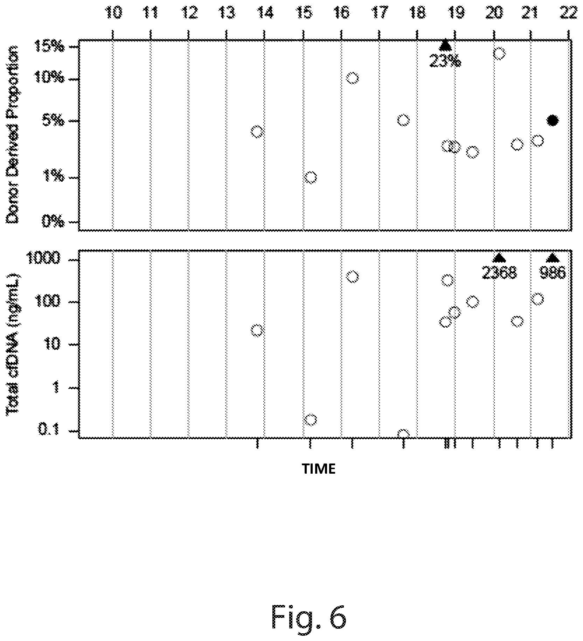

In some embodiments, the diagnostic facility may be configured to output for presentation to a user a user interface that includes a diagnosis of a risk and/or a basis for the diagnosis for a subject 802. The basis for the diagnosis may include, for example, amounts of cell-free nucleic acids (such as DNA) detected in one or more samples 806 for a subject 802. In some embodiments, user interfaces may include any of the examples of results, values, amounts, graphs, etc. discussed above. They can include results, values, amounts, etc. over time. For example, in some embodiments, a user interface may incorporate a graph similar to that shown in **Fig. 6** and/or **Fig. 7** and/or **Fig. 9.** In such a case, in some cases the graph may be annotated to indicate to a user how different regions of the graph may correspond to different diagnoses that may be produced from an analysis of data displayed in the graph. For example, thresholds against which the graphed data may be compared to determine the analysis may be imposed on the graph(s). In the case of the graph of **Fig. 7** or **Fig. 9,** this may include adding two lines to the graph, separating the graph into four quadrants, with one line indicating a threshold on the x-axis and another line indicating a threshold on the y-axis. In some embodiments, the quadrants may additionally or alternatively be shaded, such as with shading of different transparencies and/or colors. In embodiments in which the diagnostic facility evaluates more than two thresholds, more than four areas may be indicated through lines and/or shading.

A user interface including the graph of **Fig. 7** or **Fig. 9,** particularly with the lines and/or shading, may provide a user with a far more intuitive and faster-to-review interface to determine a risk of the subject 802 based on amounts of cell-free nucleic acids (such as DNA), than may be provided through other user interfaces. For example, a user interface based on **Fig. 7** or **Fig. 9** may be much more intuitive and faster-to-review than a user interface based solely on **Fig. 6.** As such, there may be specific and substantial benefit to a user interface using the graph of **Fig. 7** or **Fig. 9.** A user interface including the graph of **Fig. 6** and **Fig. 7** or **Fig. 9,** particularly with the lines and/or shading, may also provide a user with a far more intuitive and faster-to-review interface to determine a risk of the subject 802 based on amounts of cell-free nucleic acids (such as DNA), than may be provided through other user interfaces. The combination of **Fig. 6** and **Fig. 7** or **Fig. 9** may be much more intuitive and faster-to-review. As such, there may be specific and substantial benefit to a user interface using both graphs. It should be appreciated, however, that embodiments are not limited to being implemented with any particular user interface.

In some embodiments, the diagnostic facility may output the diagnosis or a user interface to one or more other computing devices 814 (including devices 814A, 814B) that may be operated by the subject 802 and/or a clinician, which may be the clinician 804 or another clinician. The diagnostic facility may transmit the diagnosis and/or user interface to the device 814 via the network(s) 812.

Techniques operating according to the principles described herein may be implemented in any suitable manner. Included in the discussion above are a series of flow charts showing the steps and acts of various processes that determine a risk of a condition based on an analysis of amounts of cell-free nucleic acids (such as DNA). The processing and decision blocks discussed above represent steps and acts that may be included in algorithms that carry out these various processes. Algorithms derived from these processes may be implemented as software integrated with and directing the operation of one or more single- or multi-purpose processors, may be implemented as functionally-equivalent circuits such as a Digital Signal Processing (DSP) circuit or an Application-Specific Integrated Circuit (ASIC), or may be implemented in any other suitable manner. It should be appreciated that embodiments are not limited to any particular syntax or operation of any particular circuit or of any particular programming language or type of programming language. Rather, one skilled in the art may use the description above to fabricate circuits or to implement computer software algorithms to perform the processing of a particular apparatus carrying out the types of techniques described herein. It should also be appreciated that, unless otherwise indicated herein, the particular sequence of steps and/or acts described above is merely illustrative of the algorithms that may be implemented and can be varied in implementations and embodiments of the principles described herein.

Accordingly, in some embodiments, the techniques described herein may be embodied in computer-executable instructions implemented as software, including as application software, system software, firmware, middleware, embedded code, or any other suitable type of computer code. Such computer-executable instructions may be written using any of a number of suitable programming languages and/or programming or scripting tools, and also may be compiled as executable machine language code or intermediate code that is executed on a framework or virtual machine.

When techniques described herein are embodied as computer-executable instructions, these computer-executable instructions may be implemented in any suitable manner, including as a number of functional facilities, each providing one or more operations to complete execution of algorithms operating according to these techniques. A "functional facility," however instantiated, is a structural component of a computer system that, when integrated with and executed by one or more computers, causes the one or more computers to perform a specific operational role. A functional facility may be a portion of or an entire software element. For example, a functional facility may be implemented as a function of a process, or as a discrete process, or as any other suitable unit of processing. If techniques described herein are implemented as multiple functional facilities, each functional facility may be implemented in its own way; all need not be implemented the same way. Additionally, these functional facilities may be executed in parallel and/or serially, as appropriate, and may pass information between one another using a shared memory on the computer(s) on which they are executing, using a message passing protocol, or in any other suitable way.

Generally, functional facilities include routines, programs, objects, components, data structures, etc. that perform particular tasks or implement particular abstract data types. Typically, the functionality of the functional facilities may be combined or distributed as desired in the systems in which they operate. In some implementations, one or more functional facilities carrying out techniques herein may together form a complete software package. These functional facilities may, in alternative embodiments, be adapted to interact with other, unrelated functional facilities and/or processes, to implement a software program application.

Some exemplary functional facilities have been described herein for carrying out one or more tasks. It should be appreciated, though, that the functional facilities and division of tasks described is merely illustrative of the type of functional facilities that may implement the exemplary techniques described herein, and that embodiments are not limited to being implemented in any specific number, division, or type of functional facilities. In some implementations, all functionality may be implemented in a single functional facility. It should also be appreciated that, in some implementations, some of the functional facilities described herein may be implemented together with or separately from others (i.e., as a single unit or separate units), or some of these functional facilities may not be implemented.

Computer-executable instructions implementing the techniques described herein (when implemented as one or more functional facilities or in any other manner) may, in some embodiments, be encoded on one or more computer-readable media to provide functionality to the media. Computer-readable media include magnetic media such as a hard disk drive, optical media such as a Compact Disk (CD) or a Digital Versatile Disk (DVD), a persistent or non-persistent solid-state memory (e.g., Flash memory, Magnetic RAM, etc.), or any other suitable storage media. Such a computer-readable medium may be implemented in any suitable manner, including as a portion of a computing device or as a stand-alone, separate storage medium. As used herein, "computer-readable media" (also called "computer-readable storage media") refers to tangible storage media. Tangible storage media are non-transitory and have at least one physical, structural component. In a "computer-readable medium," as used herein, at least one physical, structural component has at least one physical property that may be altered in some way during a process of creating the medium with embedded information, a process of recording information thereon, or any other process of encoding the medium with information. For example, a magnetization state of a portion of a physical structure of a computer-readable medium may be altered during a recording process.

In some, but not all, implementations in which the techniques may be embodied as computer-executable instructions, these instructions may be executed on one or more suitable computing device(s) operating in any suitable computer system, including the exemplary computer system of **Fig. 8,** or one or more computing devices (or one or more processors of one or more computing devices) may be programmed to execute the computer-executable instructions. A computing device or processor may be programmed to execute instructions when the instructions are stored in a manner accessible to the computing device or processor, such as in a data store (e.g., an on-chip cache or instruction register, a computer-readable storage medium accessible via a bus, etc.). Functional facilities comprising these computer-executable instructions may be integrated with and direct the operation of a single multi-purpose programmable digital computing device, a coordinated system of two or more multi-purpose computing device sharing processing power and jointly carrying out the techniques described herein, a single computing device or coordinated system of computing device (co-located or geographically distributed) dedicated to executing the techniques described herein, one or more Field-Programmable Gate Arrays (FPGAs) for carrying out the techniques described herein, or any other suitable system.

Embodiments have been described where the techniques are implemented in circuitry and/or computer-executable instructions. It should be appreciated that some embodiments may be in the form of a method, of which at least one example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments. Any one of the aforementioned, including the aforementioned devices, systems, embodiments, methods, techniques, algorithms, media, hardware, software, interfaces, processors, displays, networks, inputs, outputs or any combination thereof are provided herein in other aspects.

**Claims**

1. A method of assessing one or more samples from a subject who is a transplant recipient, wherein the one or more samples comprise a blood, plasma or serum sample obtained from the subject, comprising:

> determining a value for the amount of total cell-free DNA in one or more samples from the subject, and
> determining a value for the amount of donor specific cell-free DNA in one or more samples from the subject,
> wherein the method further comprises determining transplant rejection risk in the subject using the value for the amount of total cell-free DNA and the value for the amount of donor specific cell-free DNA, wherein the subject is identified as having transplant rejection risk when the value for the amount of total cell-free DNA exceeds a threshold value of 15 ng/ml plasma or more and
> the value for the amount of donor specific cell-free DNA exceeds a threshold value of 0.8% or more.

2. The method of claim 1, wherein the method further comprises:
providing the value for the amount of total cell-free DNA in the one or more samples from the subject and the value for the amount of donor specific cell-free DNA in the one or more samples from the subject, optionally wherein the value for the amount of total cell-free DNA and value for the amount of donor specific cell-free DNA are provided in a report.

3. The method of claim 1 or claim 2, wherein

> (i) the value for the amount of total cell-free DNA and value for the amount of donor specific cell-free DNA is determined in one or more samples taken within 24 hours of a surgery, wherein the surgery is a transplant surgery; and/or
> (ii) the value for the amount of total cell-free DNA and value for the amount of donor specific cell-free DNA is determined in one or more samples taken within 24 hours of cross-clamp removal.

4. The method of any of the preceding claims, further comprising:

> determining a value for the amount of total cell-free DNA in one or more other samples from the subject, and
> determining a value for the amount of donor specific cell-free DNA in one or more other samples from the subject, wherein the one or more other samples are from a subsequent time point;
> optionally wherein the subsequent time point is at least one week later, at least two weeks later or wherein the subsequent time point is a month later.

5. The method of claim 4, further comprising:

> determining a value for the amount of total cell-free DNA in one or more further samples from the subject, and
> determining a value for the amount of donor specific cell-free DNA in one or more further samples from the subject, wherein the one or more further samples are from one or more other subsequent time points;
> optionally wherein the one or more other subsequent time points are at one-week intervals, at two-week intervals or at monthly intervals.

6. The method of claim 4 or claim 5, wherein the values for the amounts of total cell-free DNA and values for the amounts of donor specific cell-free DNA are provided in a report; optionally wherein the values for the amounts of total cell-free DNA and values for the amounts of donor specific cell-free DNA are provided separately in the report, optionally wherein the values for the amounts of total cell-free DNA and values for the amounts of donor specific cell-free DNA are provided in separate graphs showing the values for the amounts of each over time; optionally wherein the values for the amounts of total cell-free DNA and values for the amounts of donor specific cell-free DNA are provided together in the report, optionally wherein the values for the amounts of total cell-free DNA and values for the amounts of donor specific cell-free DNA are provided in the same graph in the report, optionally wherein the graph is a scatter plot; or optionally wherein the values for the amounts of total cell-free DNA and values for the amounts of donor specific cell-free DNA are provided separately and together in the report, optionally wherein the values for the amounts of total cell-free DNA and values for the amounts of donor specific cell-free DNA are provided in separate graphs showing the values for the amounts of each over time and the values for the amounts of total cell-free DNA and values for the amounts of donor specific cell-free DNA are provided together in another graph in the report, optionally wherein the another graph is a scatter plot.

7. The method of any one of the preceding claims, wherein:
the amount of total cell-free DNA is measured using PCR, such as real-time PCR; and/or the amount of donor specific cell-free DNA is measured using a next-generation sequencing method.

8. The method of any one of the preceding claims, wherein the amount of donor specific cell-free DNA is measured using a mismatch amplification method.

9. A method of assessing transplant rejection risk in a subject who is a transplant recipient, wherein the one or more samples comprise a blood, plasma or serum sample obtained from the subject, comprising:

    obtaining a value for the amount of total cell-free DNA in one or more samples from the subject,
    obtaining a value for the amount of donor specific cell-free DNA in one or more samples from the subject, and
    assessing the transplant rejection risk in the subject using the value for the amount of total cell-free DNA and the value for the amount of donor specific cell-free DNA, wherein the subject is identified as having transplant rejection risk when the value for the amount of total cell-free DNA exceeds a threshold value of 15 ng/ml plasma or more and the value for the amount of donor specific cell-free DNA exceeds a threshold value of 0.8% or more.

10. The method of claim 9, wherein the method further comprises:

    providing the one or more samples from the subject;
    optionally wherein the one or more samples are from the subject within 24 hours of a surgery, and optionally wherein the surgery is a transplant surgery.

11. The method of claim 9 or claim 10, wherein the one or more samples are from the subject within 24 hours of cross-clamp removal; and/or wherein the value for the amount of total cell-free DNA and value for the amount of donor specific cell-free DNA are obtained from a report.

12. The method of any of claims 9-11, further comprising:

    obtaining a value for the amount of total cell-free DNA in one or more other samples from the subject, and
    obtaining a value for the amount of donor specific cell-free DNA in one or more other samples from the subject, wherein the one or more other samples are from a subsequent time point;
    optionally wherein the subsequent time point is at least one week later, at least two weeks later, or wherein the subsequent time point is a month later.

13. The method of claim 12, further comprising:

    obtaining a value for the amount of total cell-free DNA in one or more further samples from the subject, and
    obtaining a value for the amount of donor specific cell-free DNA in one or more further samples from the subject, wherein the one or more further samples are from one or more other subsequent time points;
    optionally wherein

        (i) the one or more other subsequent time points are at one-week intervals;
        (ii) the one or more other subsequent time points are at two-week intervals; or
        (iii) the one or more other subsequent time points are at monthly intervals.

14. The method of claim 12 or claim 13, wherein the values for the amounts of total cell-free DNA and the values for the amounts of donor specific cell-free DNA are obtained from a report; optionally wherein:

    (i) wherein the values for the amounts of total cell-free DNA and values for the amounts of donor specific cell-free DNA are provided separately in the report; optionally wherein the values for the amounts of total cell-free DNA and values for the amounts of donor specific cell-free DNA are provided in separate graphs showing the values for the amounts of each over time;
    (ii) the values for the amounts of total cell-free DNA and values for the amounts of donor specific cell-free DNA are provided together in the report, optionally wherein the values for the amounts of total cell-free DNA and values for the amounts of donor specific cell-free DNA are provided in the same graph in the report and optionally wherein the graph is a scatter plot; or
    (iii) the values for the amounts of total cell-free DNA and values for the amounts of donor specific cell-free DNA are provided separately and together in the report, optionally wherein the values for the amounts of total cell-free DNA and values for the amounts of donor specific cell-free DNA are provided in separate graphs showing the values for the amounts of each over time and the values for the amounts of total cell-free DNA and values for the amounts of donor specific cell-free DNA are provided together in another graph in the report, and optionally wherein the another graph is a scatter plot.

15. The method of any one of the preceding claims, wherein the transplant recipient is a cardiac transplant recipient, and optionally wherein the subject is a pediatric cardiac transplant recipient.

16. The method of any one of the preceding claims, wherein:

(i) when the value for the amount of total cell-free DNA is above a threshold value and the value for the amount of donor specific cell-free DNA is above a threshold value, rejection or adverse outcome or prognosis is indicated;
(ii) when the value for the amount of total cell-free DNA is above a threshold value and the value for the amount of donor specific cell-free DNA is below a threshold value, infection is indicated;
(iii) when the value for the amount of total cell-free DNA is below a threshold value and the value for the amount of donor specific cell-free DNA is above a threshold value, early stage rejection is indicated;
(iv) when the value for the amount of total cell-free DNA is below a threshold value and the value for the amount of donor specific cell-free DNA is below a threshold value, no clinical condition is indicated; and/or
(v) when the value for the amount of total cell-free DNA is below a threshold value and the value for the amount of donor specific cell-free DNA is below a threshold value, further monitoring of the subject is performed or suggested to the subject, optionally wherein the monitoring of the subject comprises the method of any one of claims 1-15.

**Patentansprüche**

1. Verfahren zum Bewerten einer oder mehrerer Proben von einem Subjekt, das ein Transplantatempfänger ist, wobei die eine oder mehreren Proben eine von dem Subjekt erlangte Blut-, Plasma- oder Serumprobe umfassen, umfassend:

Bestimmen eines Werts für die Menge an gesamter zellfreier DNA in einer oder mehreren Proben von dem Subjekt und
Bestimmen eines Werts für die Menge an spenderspezifischer zellfreier DNA in einer oder mehreren Proben von dem Subjekt,
wobei das Verfahren ferner Bestimmen eines Transplantatabstoßungsrisikos bei dem Subjekt unter Verwendung des Werts für die Menge an gesamter zellfreier DNA und des Werts für die Menge an spenderspezifischer zellfreier DNA umfasst, wobei das Subjekt als ein Transplantatabstoßungsrisiko aufweisend identifiziert wird, wenn der Wert für die Menge an gesamter zellfreier DNA einen Schwellenwert von 15 ng/ml Plasma oder mehr überschreitet und der Wert für die Menge an spenderspezifischer zellfreier DNA einen Schwellenwert von 0,8 % oder mehr überschreitet.

2. Verfahren nach Anspruch 1, wobei das Verfahren ferner Folgendes umfasst:
Bereitstellen des Werts für die Menge an gesamter zellfreier DNA in der einen oder den mehreren Proben von dem Subjekt und des Werts für die Menge an spenderspezifischer zellfreier DNA in der einen oder den mehreren Proben von dem Subjekt, wobei optional der Wert für die Menge an gesamter zellfreier DNA und der Wert für die Menge an spenderspezifischer zellfreier DNA in einem Bericht bereitgestellt werden.

3. Verfahren nach Anspruch 1 oder Anspruch 2, wobei

(i) der Wert für die Menge an gesamter zellfreier DNA und der Wert für die Menge an spenderspezifischer zellfreier DNA in einer oder mehreren Proben bestimmt werden, die innerhalb von 24 Stunden nach einer Operation entnommen werden, wobei die Operation eine Transplantationsoperation ist; und/oder
(ii) der Wert für die Menge an gesamter zellfreier DNA und der Wert für die Menge an spenderspezifischer zellfreier DNA in einer oder mehreren Proben bestimmt werden, die innerhalb von 24 Stunden nach Entfernung einer Kreuzklemme entnommen werden.

4. Verfahren nach einem der vorhergehenden Ansprüche, ferner umfassend:

Bestimmen eines Werts für die Menge an gesamter zellfreier DNA in einer oder mehreren anderen Proben von dem Subjekt und
Bestimmen eines Werts für die Menge an spenderspezifischer zellfreier DNA in einer oder mehreren anderen Proben von dem Subjekt,

wobei die eine oder mehreren anderen Proben von einem nachfolgenden Zeitpunkt stammen;

wobei optional der nachfolgende Zeitpunkt mindestens eine Woche später, mindestens zwei Wochen später ist oder wobei der nachfolgende Zeitpunkt einen Monat später ist.

5. Verfahren nach Anspruch 4, ferner umfassend:

Bestimmen eines Werts für die Menge an gesamter zellfreier DNA in einer oder mehreren weiteren Proben von dem Subjekt und

Bestimmen eines Werts für die Menge an spenderspezifischer zellfreier DNA in einer oder mehreren weiteren Proben von dem Subjekt,

wobei die eine oder mehreren weiteren Proben von einem oder mehreren anderen nachfolgenden Zeitpunkten stammen;

wobei optional der eine oder mehreren anderen nachfolgenden Zeitpunkte in Intervallen von einer Woche, in Intervallen von zwei Wochen oder in monatlichen Intervallen liegen.

6. Verfahren nach Anspruch 4 oder Anspruch 5, wobei die Werte für die Mengen an gesamter zellfreier DNA und die Werte für die Mengen an spenderspezifischer zellfreier DNA in einem Bericht bereitgestellt werden;

wobei optional die Werte für die Mengen an gesamter zellfreier DNA und die Werte für die Mengen an spenderspezifischer zellfreier DNA in dem Bericht separat bereitgestellt werden, wobei optional die Werte für die Mengen an gesamter zellfreier DNA und die Werte für die Mengen an spenderspezifischer zellfreier DNA in separaten Schaubildern bereitgestellt werden, die die Werte für die Mengen von jedem im Lauf der Zeit zeigen;

wobei optional die Werte für die Mengen an gesamter zellfreier DNA und die Werte für die Mengen an spenderspezifischer zellfreier DNA in dem Bericht zusammen bereitgestellt werden, wobei optional die Werte für die Mengen an gesamter zellfreier DNA und die Werte für die Mengen an spenderspezifischer zellfreier DNA in dem Bericht in demselben Schaubild bereitgestellt werden, wobei optional das Schaubild ein Streudiagramm ist; oder

wobei optional die Werte für die Mengen an gesamter zellfreier DNA und die Werte für die Mengen an spenderspezifischer zellfreier DNA in dem Bericht separat und zusammen bereitgestellt werden, wobei optional die Werte für die Mengen an gesamter zellfreier DNA und die Werte für die Mengen an spenderspezifischer zellfreier DNA in separaten Schaubildern bereitgestellt werden, die die Werte für die Mengen von jedem im Lauf der Zeit zeigen, und die Werte für die Mengen an gesamter zellfreier DNA und die Werte für die Mengen an spenderspezifischer zellfreier DNA in einem anderen Schaubild in dem Bericht zusammen bereitgestellt werden, wobei optional das andere Schaubild ein Streudiagramm ist.

7. Verfahren nach einem der vorhergehenden Ansprüche, wobei:

die Menge an gesamter zellfreier DNA unter Verwendung von PCR, wie Echtzeit-PCR, gemessen wird; und/oder die Menge an spenderspezifischer zellfreier DNA unter Verwendung eines Sequenzierungsverfahrens der nächsten Generation gemessen wird.

8. Verfahren nach einem der vorhergehenden Ansprüche, wobei die Menge an spenderspezifischer zellfreier DNA unter Verwendung eines Fehlpaarungsamplifikationsverfahrens gemessen wird.

9. Verfahren zum Bewerten eines Transplantatabstoßungsrisikos bei einem Subjekt, das ein Transplantatempfänger ist, wobei die eine oder mehreren Proben eine von dem Subjekt erlangte Blut-, Plasma- oder Serumprobe umfassen, umfassend:

Erlangen eines Werts für die Menge an gesamter zellfreier DNA in einer oder mehreren Proben von dem Subjekt, Erlangen eines Werts für die Menge an spenderspezifischer zellfreier DNA in einer oder mehreren Proben von dem Subjekt und

Bewerten des Transplantatabstoßungsrisikos bei dem Subjekt unter Verwendung des Werts für die Menge an gesamter zellfreier DNA und des Werts für die Menge an spenderspezifischer zellfreier DNA, wobei das Subjekt als ein Transplantatabstoßungsrisiko aufweisend identifiziert wird, wenn der Wert für die Menge an gesamter zellfreier DNA einen Schwellenwert von 15 ng/ml Plasma oder mehr überschreitet und der Wert für die Menge an spenderspezifischer zellfreier DNA einen Schwellenwert von 0,8 % oder mehr überschreitet.

**10.** Verfahren nach Anspruch 9, wobei das Verfahren ferner Folgendes umfasst:

Bereitstellen der einen oder der mehreren Proben von dem Subjekt;
wobei optional die eine oder mehreren Proben von dem Subjekt innerhalb von 24 Stunden nach einer Operation stammen und wobei optional die Operation eine Transplantationsoperation ist.

**11.** Verfahren nach Anspruch 9 oder Anspruch 10, wobei die eine oder mehreren Proben von dem Subjekt innerhalb von 24 Stunden nach Entfernung einer Kreuzklemme stammen; und/oder wobei der Wert für die Menge an gesamter zellfreier DNA und der Werte für die Menge an spenderspezifischer zellfreier DNA aus einem Bericht erlangt werden.

**12.** Verfahren nach einem der Ansprüche 9-11, ferner umfassend:

Erlangen eines Werts für die Menge an gesamter zellfreier DNA in einer oder mehreren anderen Proben von dem Subjekt und
Erlangen eines Werts für die Menge an spenderspezifischer zellfreier DNA in einer oder mehreren anderen Proben von dem Subjekt,
wobei die eine oder mehreren anderen Proben von einem nachfolgenden Zeitpunkt stammen;
wobei optional der nachfolgende Zeitpunkt mindestens eine Woche später, mindestens zwei Wochen später ist oder wobei der nachfolgende Zeitpunkt einen Monat später ist.

**13.** Verfahren nach Anspruch 12, ferner umfassend:

Erlangen eines Werts für die Menge an gesamter zellfreier DNA in einer oder mehreren weiteren Proben von dem Subjekt und
Erlangen eines Werts für die Menge an spenderspezifischer zellfreier DNA in einer oder mehreren weiteren Proben von dem Subjekt,
wobei die eine oder mehreren weiteren Proben von einem oder mehreren anderen nachfolgenden Zeitpunkten stammen;
wobei optional

(i) der eine oder die mehreren anderen nachfolgenden Zeitpunkte in Intervallen von einer Woche liegen;
(ii) der eine oder die mehreren anderen nachfolgenden Zeitpunkte in Intervallen von zwei Wochen liegen; oder
(iii) der eine oder die mehreren anderen nachfolgenden Zeitpunkte in monatlichen Intervallen liegen.

**14.** Verfahren nach Anspruch 12 oder Anspruch 13, wobei die Werte für die Mengen an gesamter zellfreier DNA und die Werte für die Mengen an spenderspezifischer zellfreier DNA aus einem Bericht erlangt werden; wobei optional:

(i) wobei die Werte für die Mengen an gesamter zellfreier DNA und die Werte für die Mengen an spenderspezifischer zellfreier DNA in dem Bericht separat bereitgestellt werden; wobei optional die Werte für die Mengen an gesamter zellfreier DNA und die Werte für die Mengen an spenderspezifischer zellfreier DNA in separaten Schaubildern bereitgestellt werden, die die Werte für die Mengen von jedem im Lauf der Zeit zeigen;
(ii) die Werte für die Mengen an gesamter zellfreier DNA und die Werte für die Mengen an spenderspezifischer zellfreier DNA in dem Bericht zusammen bereitgestellt werden, wobei optional die Werte für die Mengen an gesamter zellfreier DNA und die Werte für die Mengen an spenderspezifischer zellfreier DNA in dem Bericht in demselben Schaubild bereitgestellt werden und wobei optional das Schaubild ein Streudiagramm ist; oder
(iii) die Werte für die Mengen an gesamter zellfreier DNA und die Werte für die Mengen an spenderspezifischer zellfreier DNA in dem Bericht separat und zusammen bereitgestellt werden, wobei optional die Werte für die Mengen an gesamter zellfreier DNA und die Werte für die Mengen an spenderspezifischer zellfreier DNA in separaten Schaubildern bereitgestellt werden, die die Werte für die Mengen von jedem im Lauf der Zeit zeigen, und die Werte für die Mengen an gesamter zellfreier DNA und die Werte für die Mengen an spenderspezifischer zellfreier DNA in einem anderen Schaubild in dem Bericht zusammen bereitgestellt werden und wobei optional das andere Schaubild ein Streudiagramm ist.

**15.** Verfahren nach einem der vorhergehenden Ansprüche, wobei der Transplantatempfänger ein Herztransplantatempfänger ist und wobei optional das Subjekt ein pädiatrischer Herztransplantatempfänger ist.

**16.** Verfahren nach einem der vorhergehenden Ansprüche, wobei:

(i) wenn der Wert für die Menge an gesamter zellfreier DNA über einem Schwellenwert liegt und der Wert für die Menge an spenderspezifischer zellfreier DNA über einem Schwellenwert liegt, eine Abstoßung oder ein nachteiliges Ergebnis oder eine nachteilige Prognose angegeben wird;
(ii) wenn der Wert für die Menge an gesamter zellfreier DNA über einem Schwellenwert liegt und der Wert für die Menge an spenderspezifischer zellfreier DNA unter einem Schwellenwert liegt, eine Infektion angegeben wird;
(iii) wenn der Wert für die Menge an gesamter zellfreier DNA unter einem Schwellenwert liegt und der Wert für die Menge an spenderspezifischer zellfreier DNA über einem Schwellenwert liegt, eine Abstoßung im Frühstadium angegeben wird;
(iv) wenn der Wert für die Menge an gesamter zellfreier DNA unter einem Schwellenwert liegt und der Wert für die Menge an spenderspezifischer zellfreier DNA unter einem Schwellenwert liegt, kein klinischer Zustand angegeben wird; und/oder
(v) wenn der Wert für die Menge an gesamter zellfreier DNA unter einem Schwellenwert liegt und der Wert für die Menge an spenderspezifischer zellfreier DNA unter einem Schwellenwert liegt, weiteres Überwachen des Subjekts durchgeführt oder dem Subjekt vorgeschlagen wird, wobei optional das Überwachen des Subjekts das Verfahren nach einem der Ansprüche 1-15 umfasst.

## Revendications

1. Procédé d'évaluation d'un ou plusieurs échantillons d'un sujet qui est un receveur de greffe, dans lequel le ou les échantillons comprennent un échantillon de sang, de plasma ou de sérum obtenu auprès du sujet, comprenant :

la détermination d'une valeur pour la quantité d'ADN acellulaire total dans un ou plusieurs échantillons du sujet, et
la détermination d'une valeur pour la quantité d'ADN acellulaire spécifique du donneur dans un ou plusieurs échantillons du sujet,
ledit procédé comprenant en outre la détermination d'un risque de rejet de greffe chez le sujet à l'aide de la valeur pour la quantité d'ADN acellulaire total et de la valeur pour la quantité d'ADN acellulaire spécifique du donneur, ledit sujet étant identifié comme comportant un risque de rejet de greffe lorsque la valeur pour la quantité d'ADN acellulaire total dépasse une valeur seuil de 15 ng/ml de plasma ou plus et que la valeur pour la quantité d'ADN acellulaire spécifique du donneur dépasse une valeur seuil de 0,8 % ou plus.

2. Procédé de la revendication 1, ledit procédé comprenant en outre :
la fourniture de la valeur pour la quantité d'ADN acellulaire total dans le ou les échantillons du sujet et de la valeur pour la quantité d'ADN acellulaire spécifique du donneur dans le ou les échantillons du sujet, éventuellement dans lequel la valeur pour la quantité d'ADN acellulaire total et la valeur pour la quantité d'ADN acellulaire spécifique du donneur sont fournies dans un rapport.

3. Procédé de la revendication 1 ou la revendication 2, dans lequel

(i) la valeur pour la quantité d'ADN acellulaire total et la valeur pour la quantité d'ADN acellulaire spécifique du donneur sont déterminées dans un ou plusieurs échantillons prélevés dans les 24 heures suivant une chirurgie, ladite chirurgie étant une chirurgie de transplantation ; et/ou
(ii) la valeur pour la quantité d'ADN acellulaire total et la valeur pour la quantité d'ADN acellulaire spécifique du donneur sont déterminées dans un ou plusieurs échantillons prélevés dans les 24 heures suivant le retrait de la pince croisée.

4. Procédé de l'une quelconque des revendications précédentes, comprenant en outre :

la détermination d'une valeur pour la quantité d'ADN acellulaire total dans un ou plusieurs échantillons supplémentaires du sujet, et
la détermination d'une valeur pour la quantité d'ADN acellulaire spécifique du donneur dans un ou plusieurs échantillons supplémentaires du sujet,
dans lequel le ou les échantillons supplémentaires proviennent d'un instant ultérieur ;
éventuellement, dans lequel l'instant ultérieur est au moins une semaine plus tard, au moins deux semaines plus tard ou dans lequel l'instant ultérieur est un mois plus tard.

5. Procédé de la revendication 4, comprenant en outre :

la détermination d'une valeur pour la quantité d'ADN acellulaire total dans un ou plusieurs échantillons supplémentaires du sujet, et

la détermination d'une valeur pour la quantité d'ADN acellulaire spécifique du donneur dans un ou plusieurs échantillons supplémentaires du sujet,

dans lequel le ou les échantillons supplémentaires proviennent d'un ou plusieurs autres instants ultérieurs ; éventuellement dans lequel le ou les autres instants ultérieurs sont à des intervalles d'une semaine, à des intervalles de deux semaines ou à des intervalles mensuels.

6. Procédé de la revendication 4 ou la revendication 5, dans lequel les valeurs pour les quantités d'ADN acellulaire total et les valeurs pour les quantités d'ADN acellulaire spécifique du donneur sont fournies dans un rapport ;

éventuellement dans lequel les valeurs pour les quantités d'ADN acellulaire total et les valeurs pour les quantités d'ADN acellulaire spécifique du donneur sont fournies séparément dans le rapport,

éventuellement dans lequel les valeurs pour les quantités d'ADN acellulaire total et les valeurs pour les quantités d'ADN acellulaire spécifique du donneur sont fournies dans des graphiques séparés montrant les valeurs pour les quantités de chacun au fil du temps ;

éventuellement dans lequel les valeurs pour les quantités d'ADN acellulaire total et les valeurs pour les quantités d'ADN acellulaire spécifique du donneur sont fournies ensemble dans le rapport,

éventuellement dans lequel les valeurs pour les quantités d'ADN acellulaire total et les valeurs pour les quantités d'ADN acellulaire spécifique du donneur sont fournies dans le même graphique dans le rapport,

éventuellement dans lequel le graphique est un diagramme de dispersion ; ou

éventuellement dans lequel les valeurs pour les quantités d'ADN acellulaire total et les valeurs pour les quantités d'ADN acellulaire spécifique du donneur sont fournies séparément et ensemble dans le rapport,

éventuellement dans lequel les valeurs pour les quantités d'ADN acellulaire total et les valeurs pour les quantités d'ADN acellulaire spécifique du donneur sont fournies dans des graphiques séparés montrant les valeurs pour les quantités de chacun au fil du temps et les valeurs pour les quantités d'ADN acellulaire total et les valeurs pour les quantités d'ADN acellulaire spécifique du donneur sont fournies ensemble dans un autre graphique dans le rapport,

éventuellement dans lequel l'autre graphique est un diagramme de dispersion.

7. Procédé de l'une quelconque des revendications précédentes, dans lequel :

la quantité d'ADN acellulaire total est mesurée à l'aide d'une PCR, telle qu'une PCR en temps réel ; et/ou

la quantité d'ADN acellulaire spécifique du donneur est mesurée à l'aide d'un procédé de séquençage de nouvelle génération.

8. Procédé de l'une quelconque des revendications précédentes, dans lequel la quantité d'ADN acellulaire spécifique du donneur est mesurée à l'aide d'un procédé d'amplification de mésappariement.

9. Procédé d'évaluation d'un risque de rejet de greffe chez un sujet qui est un receveur de greffe, dans lequel le ou les échantillons comprennent un échantillon de sang, de plasma ou de sérum obtenu auprès du sujet, comprenant :

l'obtention d'une valeur pour la quantité d'ADN acellulaire total dans un ou plusieurs échantillons du sujet,

l'obtention d'une valeur pour la quantité d'ADN acellulaire spécifique du donneur dans un ou plusieurs échantillons du sujet, et

l'évaluation du risque de rejet de greffe chez le sujet à l'aide de la valeur pour la quantité d'ADN acellulaire total et de la valeur pour la quantité d'ADN acellulaire spécifique du donneur, le sujet étant identifié comme présentant un risque de rejet de greffe lorsque la valeur pour la quantité d'ADN acellulaire total dépasse une valeur seuil de 15 ng/ml de plasma ou plus et que la valeur pour la quantité d'ADN acellulaire spécifique du donneur dépasse une valeur seuil de 0,8 % ou plus.

10. Procédé de la revendication 9, ledit procédé comprenant en outre :

la fourniture du ou des échantillons du sujet ;

éventuellement dans lequel le ou les échantillons proviennent du sujet dans les 24 heures suivant une chirurgie, et éventuellement dans lequel la chirurgie est une chirurgie de transplantation.

11. Procédé de la revendication 9 ou de la revendication 10, dans lequel le ou les échantillons proviennent du sujet dans les 24 heures suivant le retrait de la pince croisée ; et/ou
dans lequel la valeur pour la quantité d'ADN acellulaire total et la valeur pour la quantité d'ADN acellulaire spécifique du donneur sont obtenues à partir d'un rapport.

**12.** Procédé de l'une quelconque des revendications 9 à 11, comprenant en outre :

l'obtention d'une valeur pour la quantité d'ADN acellulaire total dans un ou plusieurs échantillons supplémentaires du sujet, et
l'obtention d'une valeur pour la quantité d'ADN acellulaire spécifique du donneur dans un ou plusieurs échantillons supplémentaires du sujet,
dans lequel le ou les échantillons supplémentaires proviennent d'un instant ultérieur ;
éventuellement dans lequel l'instant ultérieur est au moins une semaine plus tard, au moins deux semaines plus tard, ou dans lequel l'instant ultérieur est un mois plus tard.

**13.** Procédé de la revendication 12, comprenant en outre :

l'obtention d'une valeur pour la quantité d'ADN acellulaire total dans un ou plusieurs échantillons supplémentaires du sujet, et
l'obtention d'une valeur pour la quantité d'ADN acellulaire spécifique du donneur dans un ou plusieurs échantillons supplémentaires du sujet,
dans lequel le ou les échantillons supplémentaires proviennent d'un ou de plusieurs autres instants ultérieurs ;
éventuellement dans lequel

(i) le ou les autres instants ultérieurs sont à des intervalles d'une semaine ;
(ii) le ou les autres instants ultérieurs sont à des intervalles de deux semaines ; ou
(iii) le ou les autres instants ultérieurs sont à des intervalles mensuels.

**14.** Procédé de la revendication 12 ou la revendication 13, dans lequel les valeurs pour les quantités d'ADN acellulaire total et les valeurs pour les quantités d'ADN acellulaire spécifique du donneur sont obtenues à partir d'un rapport ; éventuellement dans lequel :

(i) dans lequel les valeurs pour les quantités d'ADN acellulaire total et les valeurs pour les quantités d'ADN acellulaire spécifique du donneur sont fournies séparément dans le rapport ; éventuellement dans lequel les valeurs pour les quantités d'ADN acellulaire total et les valeurs pour les quantités d'ADN acellulaire spécifique du donneur sont fournies dans des graphiques séparés montrant les valeurs pour les quantités de chacun au fil du temps ;
(ii) les valeurs pour les quantités d'ADN acellulaire total et les valeurs pour les quantités d'ADN acellulaire spécifique du donneur sont fournies ensemble dans le rapport, éventuellement dans lequel les valeurs pour les quantités d'ADN acellulaire total et les valeurs pour les quantités d'ADN acellulaire spécifique du donneur sont fournies dans le même graphique dans le rapport et éventuellement dans lequel le graphique est un diagramme de dispersion ; ou
(iii) les valeurs pour les quantités d'ADN acellulaire total et les valeurs pour les quantités d'ADN acellulaire spécifique du donneur sont fournies séparément et ensemble dans le rapport, éventuellement dans lequel les valeurs pour les quantités d'ADN acellulaire total et les valeurs pour les quantités d'ADN acellulaire spécifique du donneur sont fournies dans des graphiques séparés montrant les valeurs pour les quantités de chacun au fil du temps et les valeurs pour les quantités d'ADN acellulaire total et les valeurs pour les quantités d'ADN acellulaire spécifique du donneur sont fournies ensemble dans un autre graphique dans le rapport, et éventuellement dans lequel l'autre graphique est un diagramme de dispersion.

**15.** Procédé de l'une quelconque des revendications précédentes, dans lequel le receveur de greffe est un receveur de greffe cardiaque, et éventuellement dans lequel le sujet est un receveur de greffe cardiaque pédiatrique.

**16.** Procédé de l'une quelconque des revendications précédentes, dans lequel :

(i) lorsque la valeur pour la quantité d'ADN acellulaire total est supérieure à une valeur seuil et que la valeur pour la quantité d'ADN acellulaire spécifique du donneur est supérieure à une valeur seuil, le rejet ou le résultat ou le pronostic défavorable est indiqué ;
(ii) lorsque la valeur pour la quantité d'ADN acellulaire total est supérieure à une valeur seuil et que la valeur pour la quantité d'ADN acellulaire spécifique du donneur est inférieure à une valeur seuil, l'infection est indiquée ;

(iii) lorsque la valeur pour la quantité d'ADN acellulaire total est inférieure à une valeur seuil et que la valeur pour la quantité d'ADN acellulaire spécifique du donneur est supérieure à une valeur seuil, un rejet à un stade précoce est indiqué ;

(iv) lorsque la valeur pour la quantité d'ADN acellulaire total est inférieure à une valeur seuil et que la valeur pour la quantité d'ADN acellulaire spécifique du donneur est inférieure à une valeur seuil, aucun état clinique n'est indiqué ; et/ou

(v) lorsque la valeur pour la quantité d'ADN acellulaire total est inférieure à une valeur seuil et que la valeur pour la quantité d'ADN acellulaire spécifique du donneur est inférieure à une valeur seuil, une surveillance supplémentaire du sujet est effectuée ou suggérée au sujet, éventuellement dans lequel la surveillance du sujet comprend le procédé de l'une quelconque des revendications 1 à 15.

Fig. 1

Fig. 2

Percent Donor Specific cell free DNA (DScf-DNA)

Fig. 3

Percent Donor Specific Cell Free DNA measured by MOMA in plasma samples collected within 10 days of heart transplant patients

Fig. 4

**Descriptive Statistics +
inference**

| | Treatment for infection at draw? | | Null Hypothesis | Statistical Test |
|---|---|---|---|---|
| | no | yes | The mean or median is the same across NO and YES infection treatment. | |
| N | 275 | 35 | | |
| minimum | 1.27 | 4.40 | | |
| maximum | 574.76 | 599.54 | | |
| mean (SD) | 26.40 (51.04) | 115.62 (133.31) | p < 0.001 | T-Test |
| median [IQR] | 11.52 [5.99, 27.10] | 57.67 [18.31, 168.99] | p < 0.001 | Median Test |
| geometric mean (SD) | 13.21 (2.89) | 57.15 (3.74) | p < 0.001 | T-test |

Fig. 5

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62416689
- US 2017030293 W
- US 2016030313 W
- US 20150086477 A1
- US 20140242582 A

### Non-patent literature cited in the description

- *The Journal of Molecular Diagnostics*, 2016, vol. 18 (6), 890-902
- Harrison's Principle of Internal Medicine. McGraw Hill Inc.
- **E. W. MARTIN**. Remington's Pharmaceutical Sciences
- **STEIN RA**. Next-Generation Sequencing Update. *Genetic Engineering & Biotechnology News*, 01 September 2008, vol. 28 (15)
- **QUAIL, MICHAEL ; SMITH, MIRIAM E ; COUPLAND, PAUL ; OTTO, THOMAS D ; HARRIS, SIMON R ; CONNOR, THOMAS R ; BERTONI, ANNA ; SWERDLOW, HAROLD P ; GU, YONG**. A tale of three next generation sequencing platforms: comparison of Ion torrent, pacific biosciences and illumina MiSeq sequencers. *BMC Genomics*, 01 January 2012, vol. 13 (1), 341
- **LIU, LIN ; LI, YINHU ; LI, SILIANG ; HU, NI ; HE, YIMIN ; PONG, RAY ; LIN, DANNI ; LU, LIHUA ; LAW, MAGGIE**. Comparison of Next-Generation Sequencing Systems. *Journal of Biomedicine and Biotechnology*, 01 January 2012, vol. 2012, 1-11
- *Methods Mol Biol.*, 2009, vol. 578, 307-43
- **CHU T ; BUNCE K ; HOGGE WA ; PETERS DG**. A novel approach toward the challenge of accurately quantifying fetal DNA in maternal plasma. *Prenat Diagn*, 2010, vol. 30, 1226-9
- **SUZUKI N ; KAMATAKI A ; YAMAKI J ; HOMMA Y**. Characterization of circulating DNA in healthy human plasma. Clinica chimica acta. *International Journal of Clinical Chemistry*, 2008, vol. 387, 55-8
- Cell-Free Plasma DNA as a Predictor of Outcome in Severe Sepsis and Septic Shock.. *Clin. Chem.*, 2008, vol. 54, 1000-1007
- Prediction of MYCN Amplification in Neuroblastoma Using Serum DNA and Real-Time Quantitative Polymerase Chain Reaction. *JCO*, 2005, vol. 23, 5205-5210
- Circulating Nucleic Acids in Blood of Healthy Male and Female Donors. *Clin. Chem.*, 2005, vol. 51, 1317-1319
- Use of Magnetic Beads for Plasma Cell-free DNA Extraction: Toward Automation of Plasma DNA Analysis for Molecular Diagnostics.. *Clin. Chem.*, 2003, vol. 49, 1953-1955
- **CHIU RWK ; POON LLM ; LAU TK ; LEUNG TN ; WONG EMC ; LO YMD**. Effects of blood-processing protocols on fetal and total DNA quantification in maternal plasma.. *Clin Chem*, 2001, vol. 47, 1607-1613
- **SWINKELS et al.** Effects of Blood-Processing Protocols on Cell-free DNA Quantification in Plasma.. *Clinical Chemistry*, 2003, vol. 49 (3), 525-526
- **HIDESTRAND et al.** Influence of temperature during transportation on cell-free DNA analysis. *Fetal Diagn Ther*, 2012, vol. 31, 122-128